# EUROPEAN PATENT SPECIFICATION

(11) **EP 2 391 352 B1**
(45) Date of publication and mention of the grant of the patent: **01.11.2017**
(21) Application number: 10702287.3
(22) Date of filing: 29.01.2010
(51) Int. Cl.: A61K 9/20, A61P 7/02, A61K 31/727

(54) **PHARMACEUTICAL FORMS FOR THE RELEASE OF ACTIVE COMPOUNDS**
PHARMAZEUTISCHE FORMEN ZUR FREISETZUNG VON WIRKSTOFFEN
FORMES PHARMACEUTIQUES POUR LA LIBÉRATION DE COMPOSÉS ACTIFS

(30) Priority: 30.01.2009 EP 09382014
(43) Date of publication of application: 07.12.2011
(73) Proprietor: Laboratorios Farmacéuticos Rovi, S.A., 28037 Madrid (ES)
(72) Inventor: FRANCO RODRÍGUEZ, Guillermo, E-28037 Madrid (ES); GUTIERRO ADURIZ, Ibón, E-28037 Madrid (ES)
(74) Representative: Carpintero Lopez, Francisco
(86) International application number: PCT/EP2010/051127
(87) International publication number: WO 2010/086425

(56) References cited:
- WO-A-02/03960
- WO-A-2005/032703
- HOFFART ET AL: "Oral bioavailability of a low molecular weight heparin using a polymeric delivery system" JOURNAL OF CONTROLLED RELEASE, ELSEVIER, AMSTERDAM, NL, vol. 113, no. 1, 12 June 2006 (2006-06-12) , pages 38-42, XP005486107 ISSN: 0168-3659

## Description

### FIELD OF THE INVENTION

The present invention relates to pharmaceutical forms for the release of glycosaminoglycans (GAG) for absorption by mucosal route after their administration, as well as to pharmaceutical formulations containng said pharmaceutical forms and to the use of said pharmaceutical forms and said formulations for the manufacture of a medicament applicable to mucosas.

The route of administration is selected from oropharyngeal mucosa, gastrointestinal mucosa, pulmonary mucosa, nasal mucosa and vaginal mucosa.

### BACKGROUND OF THE INVENTION

A problem which has been posed in recent years in the pharmaceutical sector is that there is a great variety of active compounds which are characterized fundamentally in that they cannot be administered by oral route.

The main causes why these compounds cannot be administered by this route are:
- their rapid enzymatic and metabolic degradation,
- their chemical and biological instability,
- their low solubility in aqueous medium, or
- their limited permeability in the gastrointestinal tract.

An example of this type of active compounds presenting these problems are Glycosaminoglycan-type macromolecules (GAG), such as heparin and its derivatives, LMWH (Low Molecular Weight Heparins), pentasacharides (like fondaparinux), ULMWH (Ultra Low Molecular Weight Heparins), Chondroitin sulphate, Dermatan Sulphate, etc.

Glycosaminoglycan-type macromolecules are highly sulphated not branched polysaccharides that comprise repeating disaccharides of an uronic acid (iduronic or glucuronc) linked to an aminosugar (glucosamine or galactosamine). The building blocks of these macromolecules are as follows:

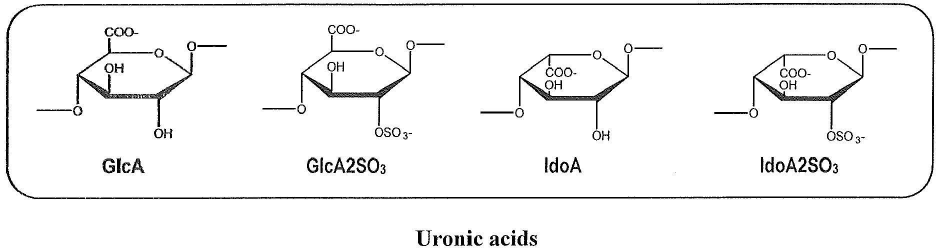

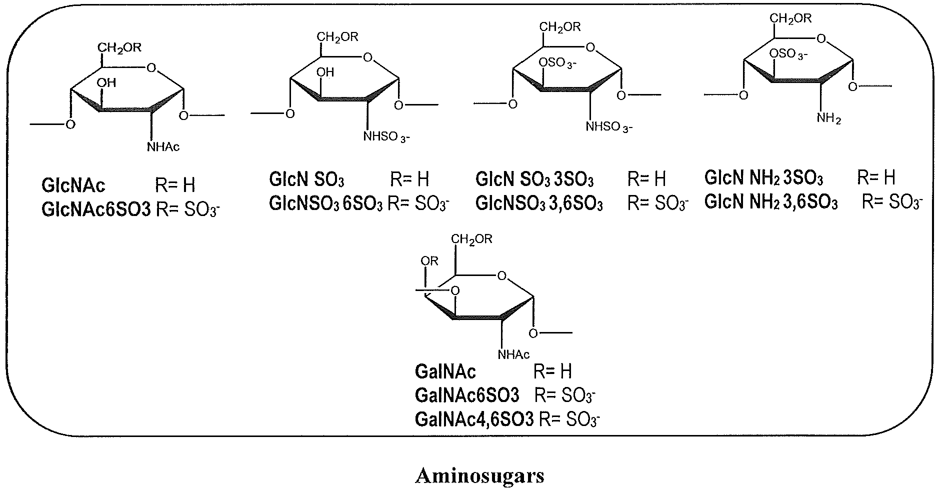

The content of sulphate and uronic groups is essential in order to evaluate the charge distribution along the polyelectrolyte chain.

Conductimetry provides a simple and rapid method for determining the absolute content of both of sulphate and carboxyl groups in glycosminoglicans (GAGs), since, conductimetric curves obtained by titrating the acid form of a GAG with a strong base are characteristic of the GAG *[*A conductimetry method for the determination of sulphate and carboxyl groupes in heparin and other muopolysaccharides. Benito Casu and Ugo Genaro. Carbohydrate Research, 39, 168-179, 1975*].* Using this methodology sulphate and carboxyl percentage in different GAGs has been determined:

| **GAG** | **SO3-% dry substance weight** | **COO-% dry substance weight** |
|---|---|---|
| Unfractionated heparin | 25-35 % | 7-8 % |
| Low Molecular Weight Heparin | 25-29 % | 6-7 % |
| Chondroitin Sulphate | 11-17 % | 6-9 % |
| Dermatan Sulphate | 15-20 % | 5-8 % |
| Fondaparinux | 37 % | 5 % |

These Glycosaminoglycan-type macromolecules are very important for treating different diseases, for example, low molecular weight heparins (LMWH) are the standard for anticoagulant used in the prevention and treatment of deep vein thrombosis and pulmonary embolism (Ageno 2000 "Treatment of venous thromboembolism." Thromb Res 97(1): V63-72; Agnelli, G. and F. Sonaglia (2000). "Prevention of venous thromboembolism." Thromb Res 97(1): V49-62.). For many years, LMWH have overcame unfractionated heparin (UFH) in many countries due to the decrease of health care cost and their simple utilization (Kakkar, A. K. (2004). "Low- and ultra-low-molecular-weight heparins." Best Pract Res Clin Haematol 17(1): 77-87). Indeed, LMWH have better predictability of the pharmacodynamic effect, an excellent bioavailability after s.c. administration and a lower risk of heparin induced thrombocytopenia (Boneu, B. (2000). "Low molecular weight heparins: are they superior to unfractionated heparins to prevent and to treat deep vein thrombosis?" Thromb Res 100(2): V113-20). "Low molecular weight heparins: are they superior to unfractionated heparins to prevent and to treat deep vein thrombosis?" Thromb Res 100(2): V113-20; Gerotziafas, G. T., A. D. Petropoulou, et al. (2007). "Effect of the anti-factor Xa and anti-factor IIa activities of low-molecular-weight heparins upon the phases of thrombin generation." J Thromb Haemost 5(5): 955-62). Unfortunately, their parenteral administration is a main disadvantage especially in the long term outpatient therapy.

Therefore, the oral dosage forms would be a major improvement in the administration of said LMWH, since it represents the most convenient and physiological route. Despite the strong anionic charge and important molecular size of LMWH, researchers have tried for many years to develop oral formulation of these macromolecules (Jaques Lb, 1979 "Heparins - Anionic Poly-Electrolyte Drugs" Pharmacol. Rev., 68).

In fact, heparins are glycosaminoglycan macromolecules composed of a mixture of different chains lengths: their composition is very heterogeneous (Hemker, H. C. and S. Beguin, Eds. (1994). Low-Molecular-Weight Heparins in Prophylaxis and Therapy of Thromboembolic Diseases. Fundamental clinical cardiology Editions Medicales Internationales). Low-Molecular-Weight Heparins in Prophylaxis and Therapy of Thromboembolic Diseases. Fundamental clinical cardiology Editions Medicales Internationales). They act by linking to antithrombin, a natural inhibitor of the coagulation cascade. The capacity of heparin to bind and catalyze the antithrombin (AT) action is selectively supported by a specific pentasacharidic structure, present only in one quarter of the total population chains (Choay, J. (1986). "Une nouvelle approche de la chimie de l'héparine et de ses fragments : structure et synthèse." angéiologie 38(4): 123-132). The heparin-AT complex has an equimolar inhibition action on either the Xa or the IIa factor of the coagulation cascade depending on the length of the chain of heparin which presents the pentasacharidic structure.

The document "Review of bemiparin sodium, a new second, generation low molecular weight heparin and its applications in venous thromboembolism" by Andre Planès, Expert Opin. Pharmacother (2003) 4(9):1551-1561 describes bemiparin sodium. Bemiparin has the lowest mean molecular weight (3600 Da), the longest half life (5.3 h) and the largest antifactor Xa:antifactor IIa ratio (8:1) of all LMWHs.

It is necessary to consider that, although some of these compounds can be administered by oral route, they have very low and/or very erratic gastrointestinal absorption, which poses a serious problem for marketing this type of drugs, since the results obtained differ greatly from those expected due to this behaviour in the LADME pharmacokinetic processes (Liberation, Absorption, Distribution, Metabolization and Elimination). Furthermore, these macromolecules have a hydrophilic character and are very poorly permeable, which conditions a low absorption by mucosal routes which, in the case of the oral route, is worsened as these macromolecules have to pass through the stomach.

Like any cellular structure, the digestive mucosa easily allows small lipophilic molecules pass through, but it is very impermeable to charged hydrophilic macromolecules. Furthermore, this aspect is amplified by the fact that the digestive tube is coated with negatively charged mucosa which has a natural tendency to repel the molecules of the same charge.

A clear example of an active compound whose absorption is limited by oral route is heparin, since following its administration by oral route, the absorption through the digestive mucosa will not be limited by its solubility, but by its low permeability with respect to the intestinal membrane, mainly due to its negative charge. The permeability of heparins through the digestive epithelium is, therefore, very low, which makes the administration of heparin/formulations containing heparin by oral route very difficult.

For this reason, the route of administration chosen in the majority of the aforementioned classes of compounds is the route of parenteral administration, mainly via intramuscular or subcutaneous injections. At present, some of these compounds are even administered via nasal and pulmonary formulations as in the case of salmon calcitonin or insulin *[*Alpar, H. J.; Somavarapu, S; Atuah, K. N.; Bramwell, V. W. Adv. Drug Deliv. Rev. 2005, 57, 411-430*; Paltz, R. M.; Patton, J. S; Foster, L.; Mohammed, E. USA Appl. No* 355578*].*

Nevertheless, a problem posed by these routes of administration is that, in most cases, these treatments require long periods of therapy such as, for example, in some types of diabetes, for which treatment is needed for the entire life, the frequency of administration being daily. This entails a great disadvantage, mainly for the patient, and for this reason it is important to look for alternative routes. Fundamentally, the oral route, that is the most convenient for the patient and the most economical, should correspond to the preferred one. However, for this type of molecules (mainly for oligosaccharides and proteins), designing formulations prepared to be administered by oral route entails a problem and involves many complications, since the gastrointestinal tract degrades these active compounds. This means that they should be formulated to enable, first, the pharmaceutical form to pass through the stomach without degrading the active compound and once it reaches the optimum absorption mucosa, release a large quantity of this active compound selectively on the mucosa wall in a relatively short time interval, consequently, the desired therapeutic or preventive activity is obtained.

Within the lines of research developed in order to obtain vehicles designed to increase systemic bioavailability after the mucosal administration of numerous Glycosaminoglycan-type macromolecules which are traditionally poorly absorbable, the following are included:
- Design of prodrugs *[Prodrug strategies to enhance the intestinal absorption of peptides.* Gangwar et al. Drug Discovery Today (1997), 2(4), 148-155*.]*
- Design of salts of the glycosaminoglycan, with a change in the chemical structure of the glycosaminoglycan [Application patent WO2007079252].
- Development of absorption promoters *[Patent* EP1652836*; patent* IS 200602146*].*
- Development of mucoadhesive devices such as bioadhesive systems or intestinal patches *[*Oral delivery of macromolecules using intestinal patches: applications for insulin delivery. Journal of Controlled Release (2004), 98(1), 37-45*].*
- Development of particulate systems.

Current research in pharmacology (based on particulate systems) is focussed on two different but complementary areas: targeting and controlled release systems.

The release profile of the active compound depends on numerous parameters: size, distribution, porosity, degradation, permeability of the polymer, etc.

An example of oral dosage form is multiparticulate form based on a mixture of a biodegradable polymer and a non biodegradable polycationic polymer. It has obtained mean absolute bioavailabilities of 59 % and 48% with nanoparticles of LMWH (Hoffart, V., A. Lamprecht, et al. (2006). "Oral bioavailability of a low molecular weight heparin using a polymeric delivery system." J Control Release 113(1): 38-42) and microparticles of UFH (Jiao, Y., N. Ubrich, et al. (2002). "Anticoagulant activity of heparin following oral administration of heparin-loaded microparticles in rabbits." J Pharm Sci 91(3): 760-8), respectively.

Patent application US 2005/0020539 A1 discloses pharmaceutical compositions and methods of preparation for the oral administration of heparin for its selective release in the intestine, which comprises a structure of multiple matrices which comprises a) an internal matrix of amphiphilic compounds and lipophilic compounds wherein the active compound is at least partially embedded and b) an outer hydrophilic matrix wherein matrix a) is dispersed. In this document, it is always needed multiple matrices with amphiphilic compounds and lipophilic compounds in order to obtain a slow dissolution profile.

For the particles to have the desired activity, homogenous degradation should be favoured throughout the polymeric mass to achieve a suitable release, also, a suitable surface potential should be favoured so that the particles approximate the absorption mucosa. Currently the effort of large multinational companies in the pharmaceutical sector is focused on the development of colloidal systems with reduced particle size, as a strategy to increase the systemic bioavailability of active compounds.

One of the promising options for improving the oral absorption is the use of absorption enhancers like sodium N-[8(-2-hydroxybenzoyl)amino]decanoate (SNAD) designed for specific facilitation of LMWH intestinal absorption in similar fashion as sodium N-[8(-2-hydroxybenzoyl)amino]caprylate (SNAC) with UFH. Indeed the bioavailability of an oral administration of UFH together with the delivery agent SNAC in rats was 6 % (Leone-Bay, A., D. R. Paton, et al. (1998). "Acylated non-alpha-amino acids as novel agents for the oral delivery of heparin sodium, USP." J Control Release 50(1-3): 41-9).

Another promising study was based on the synthesis of new heparin derivatives by coupling LMWH with deoxycholic acid (DOCA). After oral administration of LMWH-DOCA in DMSO to mice, the bioavailability was 17.6 % (Kim, S. K., B. Vaishali, et al. (2006). "Oral delivery of chemical conjugates of heparin and deoxycholic acid in aqueous formulation." Thromb Res 117(4): 419-27).

Other publications in the state of the art are directed to obtain a sustained release form of heparin. The WO2007079252 specification relates to a salt of quaternary ammonium of an acid drug in the form of a suspension or an emulsion, suitable for parenteral administration and providing a sustained release of the drug. In one preferred embodiment, this invention is directed to a long term sustained release composition for parenteral administration. In this case the chemical structure of heparin is modified.

US 4,703,042 discloses orally active salts of polyanionic heparinic acid with selected polycationic materials. The salts are stable lipoidol "ion-pairs" which can be absorbed through the gastrointestinal wall and which slowly release heparinic acid to achieve long-lasting anticoagulant activity.

The patent application WO0203960 discloses particulate vector for improving the oral absorption of active principles formed from a polymer matrix comprising at least one biodegradable polymer combined with at least one polycationic polymer. The polycationic polymer may be a copolymer of acrylic and methacrylic acid ester such as Eudragit, the active principle may be heparin, a low molecular weight heparin or related product. In the examples of the patent application WO0203960 the type of heparin is not specified.

The patent application WO2005032703 discloses a device for the manufacture of microparticles or nanoparticles and to its use in a process for the manufacture of said particles, specifically microparticles of interferon and microparticles of nadroparin.

The document redacted by Hoffart et al. "Oral bioavailability of a low molecular weight heparin using a polymeric delivery system", Journal of Controlled Release 2006, vol. 113, n°1, pages 38-42, discloses the oral bioavailability of tinzaparin-loaded nanoparticles prepared with a blend of a polyester and a polycationic polymethacrylate.

Finally, in 1969, US 3,482,014 reported acid salts of heparin (sodium acid heparinate), in contrast to the known neutral salts of heparin, for the preparation of enteric pills or tablets.

There is still the need in the art for a pharmaceutical form for the release of glycosaminoglycans that achieves an efficient and rapid absorption by mucosal route of said glycosaminoglycans after their administration.

### BRIEF DESCRIPTION OF THE INVENTION

It has surprisingly been found by the inventors that it is possible to prepare pharmaceutical forms that achieve the fast release and ulterior absorption of glycosaminoglycans, particularly bemiparin, fondaparinux and enoxaparin and its pharmaceutically acceptable salts, by mucosal route in a rapid and effective way. In particular, it has been found that in contrast to some inventions of the prior art where the charges of these glycodaminoglycans are completely or nearly completely neutralized by the polycationic material, that when the charges of the glycosaminoglycan, , are only partially neutralized with selected cations, so that the glycosaminoglycans selected maintains certain degrees of freedom, both the release and the ulterior absorption of the glycosaminoglycanes by mucosal route, for instance in the intestine, are performed in a rapid and effective way, resembling the effect of a subcutaneaous administration. It has also been found that these pharmaceutical forms are only able to adequately perform the referred rapid and effective release and ulterior absorption of the glycosaminoglycans selected if said pharmaceutical forms present a humidity percentage lower than 10% w/w.

Accordingly, the present patent application relates to a pharmaceutical form comprising a glycosaminoglycan selected from the group consisting of: bemiparin, fondaparinux and enoxaparin and its pharmaceutically acceptable salts and a compound presenting independent or dependent pH quaternary ammonium groups, which is a structure of polymers and copolymers derived from acrylic and methacrylic acids esters, wherein:
- the proportion of ammonium groups in the pharmaceutical form is between 0.01 to 2.0 µmol ammonium/mg pharmaceutical form,
- the proportion of glycosaminoglycan in the pharmaceutical form is between 15 % to 50% w/w, and
- the pharmaceutical form presents a humidity percentage (w/w) lower than 10%, wherein the charges of the glycosaminoglycan are only partially neutralized with selected cations which are dependent or independent pH quaternary ammonium groups.

In this pharmaceutical form the charges of the glycosaminoglycan are only partially neutralized with selected cations which are the dependent or independent pH quaternary ammonium groups.

As it is illustrated in the Examples the ammonium proportion for our pharmaceutical formulation should be below 2 µmol ammonium units/mg formulation in order to obtain good results in plasma activity values. The humidity content for our pharmaceutical form below 10 % (w/w) also contributes in achieving a good mucosal absorption.

In a second aspect, the invention provides a pharmaceutical formulation for the administration of the previously described pharmaceutical forms, said formulation comprises the pharmaceutical form as defined above together with pharmaceutical excipients or carriers.

A third aspect of the invention is a pharmaceutical form or a pharmaceutical formulation as described above for use as a medicament.

### DESCRIPTION OF THE FIGURES

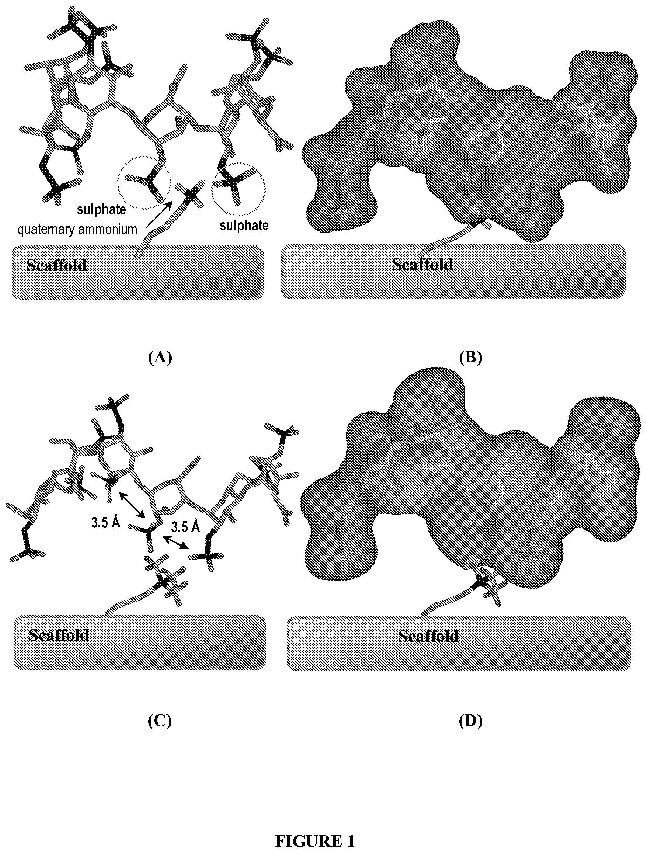

**Figure 1****:** Schematic view of the tridimensional structure of heparin-quaternary ammonium interaction. The sulphate groups close to the ammonium are represented within circles.
   A) The ammonium struture is -NH₃+
   C) The ammonium structure is -N(CH₃)₃+
   B) D) In orther to have a better picture of the interaction, the surface accesible to solvent has been represented for heparin.
**Figure 2****:** Bemiparin release profile from tablets obtained in Example 1 [compound presenting quaternary ammonium groups (Eudragit® RS PO)/glycosaminoglycan (Bemiparin)=400mg/200mg] Results are expressed as % bemiparin from tablets as function of time.
**Figure 3****:** Plasma anti Xa activity in Beagle dogs after oral administration of 20,000IU Bemiparin tablets obtained in Example 1.
**Figure 4****:** Bemiparin release profile from tablets obtained in Example 2 [compound presenting quaternary ammonium groups (Eudragit® RS PO)/glycosaminoglycan (Bemiparin)=200mg/100mg]. Results are expressed as % bemiparin from tablets as function of time.
**Figure 5****:** Plasma anti Xa activity in Beagle dogs after oral administration of 20,000IU Bemiparin in two tablets of 10,000 IU each obtained in Example 2.
**Figure 6****:** Bemiparin release profile from tablets obtained in Example 3 [compound presenting quaternary ammonium groups (Eudragit® RS PO)/glycosaminoglycan (Bemiparin)=200mg/200mg]. Results are expressed as % bemiparin from tablets as function of time.
**Figure 7****:** Plasma anti Xa activity in Beagle dogs after oral administration of 20,000IU Bemiparin tablets obtained in Example 3.
**Figure 8****:** Bemiparin release profile from tablets obtained in Example 4 [compound presenting quaternary ammonium groups (Eudragit® RS PO)/glycosaminoglycan (Bemiparin)=800mg/200mg]. Results are expressed as % bemiparin from tablets as function of time.
**Figure 9****:** Plasma anti Xa activity in Beagle dogs after oral administration of 20,000IU Bemiparin tablets obtained in Example 4.
**Figure 10****:** Bemiparin release profile from tablets obtained in Example 6 [compound presenting quaternary ammonium groups (Eudragit® RL)/glycosaminoglycan (Bemiparin)=400mg/200mg]. Results are expressed as % bemiparin from tablets as function of time.
**Figure 11****:** Plasma anti Xa activity in Beagle dogs after oral administration of 20,000IU Bemiparin tablets obtained in Example 6.
**Figure 12****:** Bemiparin release profile from tablets obtained in Example 7 [compound presenting quaternary ammonium groups (Eudragit® E)/glycosaminoglycan (Bemiparin)=400mg/200mg]. Results are expressed as % bemiparin from tablets as function of time.
**Figure 13****:** Plasma anti Xa activity in Beagle dogs after oral administration of 20,000IU Bemiparin tablets obtained in Example 7.
**Figure 14****:** Bemiparin release profile from tablets obtained in Example 8 [compound presenting quaternary ammonium groups (Eudragit® L)/glycosaminoglycan. (Bemiparin)=400mg/200mg]. Results are expressed as % bemiparin from tablets as function of time.
**Figure 15****:** Plasma anti Xa activity in Beagle dogs after oral administration of 20,000IU Bemiparin tablets obtained in Example 8.
**Figure 16****:** Bemiparin release profile from tablets obtained in Example 9 [compound presenting quaternary ammonium groups (Eudragit® RS PO)/glycosaminoglycan (Bemiparin)=5mg/2.5mg]. Results are expressed as % bemiparin from tablets as function of time.
**Figure 17****:** Plasma anti Xa activity in Beagle dogs after oral administration of 20,000IU Bemiparin tablets obtained in Example 9.
**Figure 18****:** Bemiparin release profile from tablets obtained in Example 10 [compound presenting quaternary ammonium groups (Eudragit® RS PO)/glycosaminoglycan (Bemiparin)=3.75mg/3.75mg]. Results are expressed as % bemiparin from tablets as function of time.
**Figure 19****:** Plasma anti Xa activity in Beagle dogs after oral administration of 20,000IU Bemiparin tablets obtained in Example 10.
**Figure 20****:** Bemiparin release profile from tablets obtained in Example 11 [compound presenting quaternary ammonium groups (Eudragit® RS PO)/glycosaminoglycan (Bemiparin)=6.0mg/1.5mg]. Results are expressed as % bemiparin from tablets as function of time.
**Figure 21****:** Plasma anti Xa activity in Beagle dogs after oral administration of 20,000IU Bemiparin tablets obtained in Example 11.
**Figure 22****:** Plasma anti Xa activity in wistar rats after intragastric administration of minitablets containing 2.5 mg of Fondaparinux as GAG, and 5 mg of Eudragit RS PO as compounds presenting quaternary ammonium groups attached to a non water soluble scaffold obtained in Example 12.
**Figure 23****:** Plasma anti Xa activity in Beagle dogs after oral administration of 20,000IU Bemiparin in Eudragit RS dispersion obtained in Example 13.
**Figure 24****:** Plasma anti Xa activity in Beagle dogs after oral administration of 20,000IU Bemiparin in Eudragit RS dispersion obtained in Example 14.
**Figure 25****:** Enoxaparin release profile from tablets obtained in Example 15 [compound presenting quaternary ammonium groups (Eudragit® RS PO)/glycosaminoglycan (Enoxaparin)=400mg/200mg] Results are expressed as % enoxaparin from tablets as function of time.
**Figure 26****:** Plasma anti Xa activity in Beagle dogs after oral administration of 20,000IU Enoxaparin tablets obtained in Example 15.
**Figure 27****:** Enoxaparin release profile from tablets obtained in Example 16 [compound presenting quaternary ammonium groups (Eudragit® RS PO)/glycosaminoglycan (Enoxaparin)=200mg/200mg]. Results are expressed as % enoxaparin from tablets as function of time.
**Figure 28****:** Plasma anti Xa activity in Beagle dogs after oral administration of 20,000IU Enoxaparin tablets obtained in Example 16.
**Figure 29****:** Enoxaparin release profile from tablets obtained in Example 17 [compound presenting quaternary ammonium groups (Eudragit® RS PO)/glycosaminoglycan (Enoxaparin)=5mg/2.5mg]. Results are expressed as % enoxaparin from tablets as function of time.
**Figure 30****:** Plasma anti Xa activity in Beagle dogs after oral administration of 20,000IU Enoxaparin tablets obtained in Example 17.
**Figure 31****:** Enoxaparin release profile from tablets obtained in Example 18 [compound presenting quaternary ammonium groups (Eudragit® RS PO)/glycosaminoglycan (Enoxaparin)=3.75mg/3.75mg]. Results are expressed as % enoxaparin from tablets as function of time.
**Figure 32****:** Plasma anti Xa activity in Beagle dogs after oral administration of 20,000IU Enoxaparin tablets obtained in Example 18.
**Figure 33****:** Bemiparin release profile from tablets obtained in Example 19 [compound presenting quaternary ammonium groups (Eudragit® RS PO)/glycosaminoglycan (Bemiparin)=400mg/200mg]. Results are expressed as % bemiparin from tablets as function of time.
**Figure 34****:** Plasma anti Xa activity in Beagle dogs after oral administration of 20,000IU Bemiparin tablets obtained in Example 19.
**Figure 35****:** Bemiparin release profile from tablets obtained in Example 20 [compound presenting quaternary ammonium groups (Eudragit® RS PO/Eudragit E)/glycosaminoglycan (Bemiparin)=160mg/410mg/200mg]. Results are expressed as % bemiparin from tablets as function of time.
**Figure 36****:** Plasma anti Xa activity in Beagle dogs after oral administration of 20,000IU Bemiparin tablets obtained in Example 20.

### DETAILED DESCRIPTION OF THE INVENTION

An aspect of the present patent application refers to a pharmaceutical form as described above.

As used in the description, the following terms have the meaning indicated below, unless otherwise stated:
"Pharmaceutical form" is a combination of compounds as defined in claim 1 optionally in presence of pharmaceutically acceptable ingredients. This pharmaceutical form is used to prepare the pharmaceutical formulation which is the physical form of a dose of medicament.

"Compounds with pH dependent ammonium groups" refers to compounds in which the atom of nitrogen becomes quaternary in acid enviromnents. Thus, after administration to a patient of a pharmaceutical form of the invention comprising a compound with amino, or mono or di-alkyilamino groups, these groups transform in mono or di-allkylammonium groups when the compound reaches zones of the gastrointestinal tract with an acid physiological medium.

"Compounds with pH independent ammonium groups" refers to compounds wherein the ammonium, mono, di or tri-alkylamonium groups are present in the pharmaceutical form as it is administrated to the patient.

Charges of the glycosaminoglycan "Partially neutralized" as term use in the present invention, refers to non covalent union in order to that heparin maintains certain degrees of freedom.

"Non extended release formulation" or "fast release formulation" as terms use in the present invention, refers to those formulation with a profile such us 60 % of the GAG is released during the first six hours and 80 % during 24 hours. More preferably, 80 % of the GAG is released during the first nine hours. Even more preferably, 80 % of the GAG is released during the first six hours.

As mentioned above, the pharmaceutical form of the invention comprises a glycosaminoglycan selected from the group consisting of: bemiparin, fondaparinux and enoxaparin and its phamnaceutically acceptable salts and a compound presenting independent or dependent pH quaternary ammonium groups, which is a structure of polymers and copolymers derived from acrylic and methacrylic acids esters, wherein: the proportion of ammonium groups in the pharmaceutical form is between 0.01 to 2.0 µmol ammonium/mg pharmaceutical form, the proportion of glycosaminoglycan in the pharmaceutical form is between 15 % to 50% w/w, and the pharmaceutical form presents a humidity percentage (w/w) lower than 10%, being in this pharmaceutical form the charges of the glycosaminoglycan are only partially neutralized with selected cations which are the dependent or independent pH quaternary ammonium groups.

The specific proportion of the glycosaminoglycan in the pharmaceutical form and of ammonium groups will vary depending on the glycosaminoglycan chosen, since different glycosaminoglycans will have different number of acidic groups.

As it has been mentioned in the background art section conductimetry provides a simple and rapid method for determining the absolute content of both of sulphate and carboxyl groups in glycosaminoglycans (GAGs).

In fact, the compound presenting dependent or independent pH quaternary ammonium groups can be a compound of formula (I) or (II) 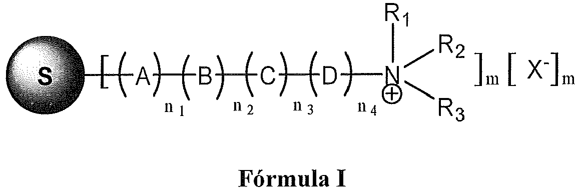 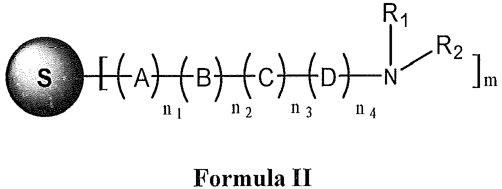 wherein: 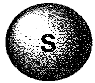 is the chemical structure or scaffold which comprises the chains -(A)-n₁-(B)n₂-(C)n₃-(D)n₄- supporting dependent or independent pH quaternary ammonium groups as part of the global structure;
m is an integer from 1 to 150
n₁, n₂, n₃ and n₄ are independent from each other: 1, 2 or 3
R₁, R₂ and R₃ are, independent from each other, a radical selected from: H, CH₃, CH₂-CH₃ and CH₂OH,
A, B, C and D are, independent from each other, a radical selected from: NH, O, S, HOP=O, O=P=O, CH₂, C=O, CH-OH, CH-NH2,
[X⁻] is a pharmaceutically acceptable anion.

Compounds of formulas (I) and (II) means a chemical structure wherein the chains -(A)-n₁-(B)n₂-(C)n₃-(D)n₄- supporting dependent or independent pH quaternary ammonium groups are anchored.

This chemical structures can correspond to structures of polymers and copolymers derived from acrylic and methacrylic acids esters (e.g., Eudragit® RS, Eudragit® RL), soluble or non soluble in water. In a particular embodiment, the structures of polymers and copolymers derived from acrylic and methacrylic acids esters is Eudragit RSPO. In another particular embodiment, the structure of polymers and copolymers derived from acrylic and methacrylic acids esters is Eurdragit RLPO:

For instance, the structure of Eudragit can be represented as follows:

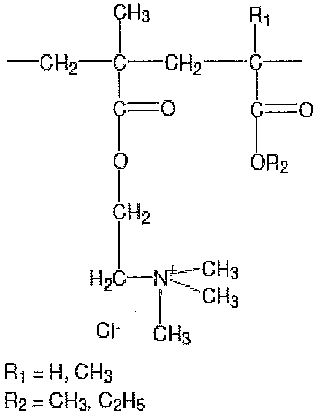

Eudragit RL 100/ RL PO and Eudragit RS 100/ RS PO are copolymers of acrylic and methacrylic acid esters with a low content in quaternary ammonium groups, Eudragit RL 100/RL PO, 8.85-11.96% ammonio methacrylate units on dry substance (DS) and Eudragit RS 100/Eudragit RS PO, 4.48-6.77% ammonio methacrylate units on DS.

The expression "pharmaceutically acceptable," as used in this description, refers to a matter which is safe and effective when it is applied in the context of the invention.

In a particular embodiment, a pharmaceutically acceptable anion [X⁻] in compounds of formula (I) and (II) is, for example, a halogen anion, more particularly, hydrochloride.

The GAG oral absorption is improved for the fact that these ammoniums are located in an structured scaffold.

According to one embodiment of the pharmaceutical form, the compound presenting dependent or independent pH quaternary ammonium groups is a non water soluble compound.

According to one embodiment of the pharmaceutical form, the proportion of ammonium groups in the pharmaceutical form is between 0.01-2 µmol ammonium/mg pharmaceutical form.

In a preferred embodiment, the proportion of ammonium groups in the pharmaceutical form is between 0.05-1.7 µmol ammonium/mg pharmaceutical form, in a more preferred embodiment, the proportion of ammonium groups in the pharmaceutical form is between 0.16-1.0 µmol ammonium/mg pharmaceutical form, and in a still more preferred embodiment the proportion of ammonium groups in the pharmaceutical form is between 0.21-0.4 µmol ammonium/mg pharmaceutical form.

According to another preferred embodiment of the pharmaceutical form, the proportion of glycosaminoglycan in the pharmaceutical form is between 15-50% w/w. In a more preferred embodiment, the proportion of glycosaminoglycan in the pharmaceutical form is between 20-50% w/w. In a still more preferred embodiment, the proportion of glycosaminoglycan in the pharmaceutical form is between 25-50% w/w, and in a even more preferred embodiment the proportion of glycosaminoglyican in the pharmaceutical form is between 33-50% w/w.

Preferably, the pharmaceutical form comprises a glycosaminoglycan and a compound presenting independent or dependent pH quaternary ammonium groups, which is a structure of polymers and copolymers derived from acrylic and methacrylic acids esters, wherein:
- the proportion of ammonium groups in the pharmaceutical form is between 0.16 to 1.7 µmol ammonium/mg pharmaceutical form,
- the proportion of glycosaminoglycan in the pharmaceutical form is between 20 % to 50% w/w, and
- the pharmaceutical form presents a humidity percentage (w/w) lower than 10%.
wherein the charges of the glycosaminoglycan are only partially neutralized with selected cations which are the dependent or independent pH quaternary ammonium groups.

More preferably, the pharmaceutical form comprises a glycosaminoglycan and a compound presenting independent or dependent pH quaternary ammonium groups, which is a structure of polymers and copolymers derived from acrylic and methacrylic acids esters, wherein:
- the proportion of ammonium groups in the pharmaceutical form is between 0.21 to 0.4 µmol ammonium/mg pharmaceutical form,
- the proportion of glycosaminoglycan in the pharmaceutical form is between 33 % to 50% w/w, and
- the pharmaceutical form presents a humidity percentage (w/w) lower than 10%.
wherein the charges of the glycosaminoglycan are only partially neutralized with selected cations which are the dependent or independent pH quaternary ammonium groups.

In another preferred embodiment, the pharmaceutical form presents a humidity percentage (w/w) lower than 10% more preferably lower than 8% (w/w) and more preferably lower than 5% (w/w).

### Among the glycosaminoglycan of interest, heparins can be mentioned.

Heparins are glycosaminoglycans (GAG) presenting anticoagulant activity which act by the inactivation of certain coagulation cascade factors. Heparins are essentially used for their anticoagulant (related with the inhibition of factor IIa) and antithrombotic (by the inhibition of factor Xa) properties for the prevention and the treatment of thromboembolic diseases *[*Low- and ultra-low--molecular-weight heparins. Best Pact Res Clin Haematol. 2004; 17: 77-87*]:*
- in prevention, to reduce the incidence of thromboembolic complications after prolonged immobilization due to a disease, and after surgical interventions *[*Prevention of venous thromboembolism. Agnelli and Sonaglia., Tromb Res. 2000, 97: V49-62*].*
- in curing, for the treatment of deep vein thrombosis *[*Treatment of venous thromboembolism. Ageno. Tromb Res. 2000, 97: V63-72*.],* of pulmonary embolisms, of disseminated intravascular coagulation, acute arterial obstruction and the acute phase of myocardial infarction.

Currently, heparin is extracted from porcine or bovine intestinal mucosa *[*Heparins: all a nephrologist should know. Hetzel et al. Nephrol Dial Transplant. 2005; 20: 2036-42*]* the unfractionated heparin is a heterogeneous mixture of sulfated mucopolysaccharide chains whose molecular mass is between 3,000 and 30,000 daltons. Its average molecular mass is 15,000 Daltons, and it corresponds to a heparin molecule of around 45 osidic units *[*Molecular weight dependency of the heparin potentiated inhibition of thrombin and activated factor X. Effect of heparin neutralization in plasma. Andersson et al. Thromb Res. 1979; 15: 531-41*].*

If a chemical or enzymatic depolymerisation is performed, heparins consisting of shorter chains, and consequently, with a lower molecular mass, between 1,000 and 10,000 daltons are produced. Their average molecular mass is 4,500 daltons. These heparins, called low molecular weight heparins (LMWH), are then distinguished from unfractioned heparins (UH) by a predominantly anti-Xa activity.

Heparin acts by the intermediation of a cofactor: antithrombin III (ATIII), which is a natural plasma inhibitor of coagulation [Heparin and Low-Molecular-Weight Heparin: The Seventh ACCP Conference on Antithrombotic and Thrombolytic Therapy. Hirsh and Raschke. Chest. 2004; 126: 188S-203S] and it behaves as a catalyst with respect to ATIII. ATIII has a slow and progressive action. Once the heparin has been fixed to the ATIII by the intermediation of the pentasaccharide fragment, this action becomes immediate. This fixation causes a change in the formation of the ATIII which then permits the irreversible fixation thereof on the active site of serine proteinase-type coagulation factors (factors IIa, Xa and IXa, mainly). Then, heparin is released intact, and can then react with a new antithrombin molecule.

It should be mentioned that the pharmacodynamic effect of heparins depends on the chain length of oligosaccharides. Indeed, to inhibit the thrombin, heparin should be fixed on ATIII and on thrombin through a pentasaccharide block. On the other hand, to inhibit factor Xa, heparin should only fix to ATIII by the pentasaccharide block. Thus, the fragments with a molecular mass (MM) below 5,400 Da, i.e. 18 saccharide units, lose their capacity to be simultaneously fixed to thrombin and ATIII, and will thus have an essentially anti-Xa activity. The fragments with a MM greater than or equal to 5,400 Da will be both anti-Xa and anti-IIa. Standard heparin comprises fragments with a variable molecular mass of 2,000 to 30,000 Da, and therefore has activity on the two factors, Xa and IIa [Heparin and Low-Molecular-Weight Heparin: The Seventh ACCP Conference on Antithrombotic and Thrombolytic Therapy. Hirsh and Raschke. Chest. 2004; 126: 188S-203S]. It therefore has both an antithrombotic and an anticoagulant activity, in comparison with low molecular weight heparins, which are essentially antithrombotic agents with a predominance of anti-Xa activity. Venous thromboembolic disease continues to be responsible for important morbidity and and/or mortality. In fact, in the United States, the number of persons hospitalized for this reason is estimated at between 300,000 and 600,000 per year. Furthermore, this disease, due to the pulmonary embolism of which it is the cause, would be responsible for 50,000 *[*Development of oral heparin therapy for prophylaxis and treatment of deep venous thrombosis. Money and Gorka. Caridovasc Surg.2001; 9: 211-8*]* to 100,000 deaths per year in the United States *[*Prevention of venous thromboembolism. Agnelli and Sonaglia. Tromb Res. 2000, 97: V49-62*].*

Since heparin acts on the coagulation factors by a catalysis mechanism mediated by ATIII, the measurement of its plasma concentration does not constitute an efficient means to determine its biological activity.

The procedures used should instead reflect heparin's capacity to inhibit factors Xa and IIa. For this reason, different measurement process can be used in human beings and animals:
- The measurement of the coagulation factor activity, expressed in units of inhibition of Xa or IIa activity.
- The measurement of the haemorrhaging time determined by the activated partial thromboplastin time (aPTT). This test explores the intrinsic route of the coagulation measuring the coagulation time of decalcified and platelet-impoverished plasma in the presence of a platelet equivalent (cephalin) and calcium.
- The measurement of the prothrombin time exploring the intrinsic coagulation route; this procedure uses recalcified blood plasma in the presence of tissue thromboplastin *[*Digestive absorption of heparin with alternative formulations. Ubrich et al., 2002. S.T.P. Pharma Sciences. 2002; 12: 147-55*]*.

There is another process which only is performed in animals, and consists of measuring the variation in the size of a thrombus. This does not permit quantifying the response, but it shows if there is a proven pharmacological action of the anticoagulant.

In a particular embodiment, the glycosaminoglycan is bemiparin. In other particular embodiment, the glycosaminoglycan is enoxaparin.

Other glycosaminoglycans that could be used in the pharmaceutical forms are chondroitin, dermatan and fondaparinux and its salts, etc.

Therefore, in an embodiment of the pharmaceutical form, the glycosaminoglycan is selected from the group consisting of unfractionated heparin, low molecular weight heparin, ultralow molecular weight heparin, chondroitin, dermatan and fondaparinux and its salts.

In a particular embodiment the pharmaceutical form additionally comprises one or more pharmaceutically acceptable excipients such as, for example, plasticizers, glidants, absorption enhancers, humectants, surfactants, colouring matters, dispersants, etc, or carriers.

In a preferred embodiment, the percentage (w/w) of excipients or carriers in the formulation is equal or lower than 10%. The pharmaceutical formulation of the invention is in solid form.

The pharmaceutical form according to the present invention may be subsequently processed to obtain a pharmaceutical formulation which is the final product to be administrated to a patient. The features of the pharmaceutical formulation depend on the route of administration, particular release profiles, or other intended effects.

For example, granules and pellets can be produced and they can also be designed for the preparation of tablets and/or capsules *[*Characterization of 5-Fluorouracil Release from Hydroxypropylmethylcellulose Compression-Coated Tablets. Wu et al. Pharmaceutical Development and Technology (2007), 12(2), 203-210*],* or they can be subject to additional treatments such as polymeric coatings of granules produced to give:
- Gastro-resistance: *[*In vitro dissolution studies of sodium diclofenac coated granules with eudragit L-30D-55 by fluidized-bed system. Silva, OR. et al. Drug Development and Industrial Pharmacy (2006), 32(6), 661-667*],* such as, for example, polyvinyl acetate phthalate, hydroxypropyl methylcellulose phthalate, acrylic copolymers as methacrylic acid and methacrylic ester copolymers, hydroxypropylmethylcellulose acetate succinate, etc.,
- Mucoadhesiveness: such as, for example, chitosan.

In a preferred embodiment, the pharmaceutical formulation presents a humidity percentage (w/w) lower than 10%, more preferably lower than 8% (w/w) and more preferably lower than 5% (w/w).

In one embodiment, the compound presenting quaternary ammonium groups form part of the surface of the said formulation. It has been found that the presence of ammonium charges on the surface of a solid form improve GAG oral absorption.

Particularly the formulation is a pellet, a granule, a tablet or a minitablet

Oral administration continues being an attractive route for the release of pharmacologically active molecules. Its easy administration, the absence of pain associated to the administration, the greater acceptance by the patient, and favourable cost/benefit ratio have turned these oral formulations into the most widely used for the administration of active compounds by oral route. Therefore, in a preferred embodiment the pharmaceutical formulation of the invention is for administration by oral route. Therefore, more particularly the formulation is for administration by oral route, and in a still more particular embodiment, the formulation is a non extended release formulation.

As mentioned above, it also forms part of the invention, the pharmaceutical form or the pharmaceutical formulation as defined above for use as a medicament. Particularly the pharmaceutical form or formulation is applicable to a mucosa selected from oropharyngeal mucosa, gastrointestinal mucosa, pulmonary mucosa, nasal mucosa and vaginal mucosa.

### EXAMPLES

The following examples illustrate the invention and should not be considered in a limitative sense thereof.

### Example 1: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 400 |
| Bemiparin | 200 |
| Mg Stearate | 3 |

This Eudragit® RSPO proportion give rise to a range of 0.173-0.261 (approximately mean of 0.21) µmol ammonium units per milligram of formulation. Ammonium unit was considered as the ammonium methacrylate monomer present on Eudragit® RSPO and not as its hydrochloride salt.

Moisture content of the present tablet composition was determined by weight loss on drying at 100°C during 15min. The mean value of humidity was 5.79, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

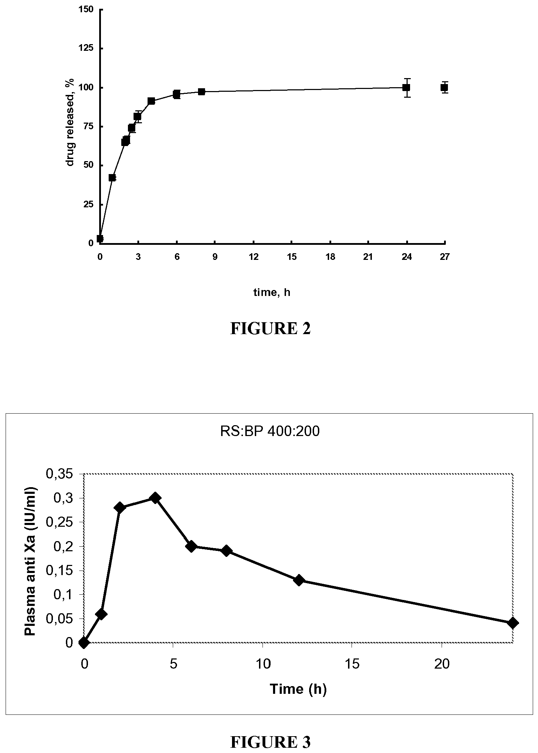

Bemiparin release profile from tablets obtained in this example is shown in Figure 2. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 3. This Eudragit® RSPO proportion give rise to a approximately 0.21 µmol ammonium units per milligram of formulation.

### Example 2: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 200 |
| Bemiparin | 100 |
| Mg Stearate | 1.5 |

This Eudragit® RSPO proportion gives rise to a range of 0.173-0.261 (approximately mean of 0.2.1) sol ammonium units per milligram of formulation. Ammonium unit was considered as the ammonium methacrylate monomer present on Eudragit® RSPO and not as its hychochloride salt.

Humidity content of the tablet corresponding to this example composition was determined by weight loss on drying at 100°C during 15min. The mean value of humidity or moisture was 4.67, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

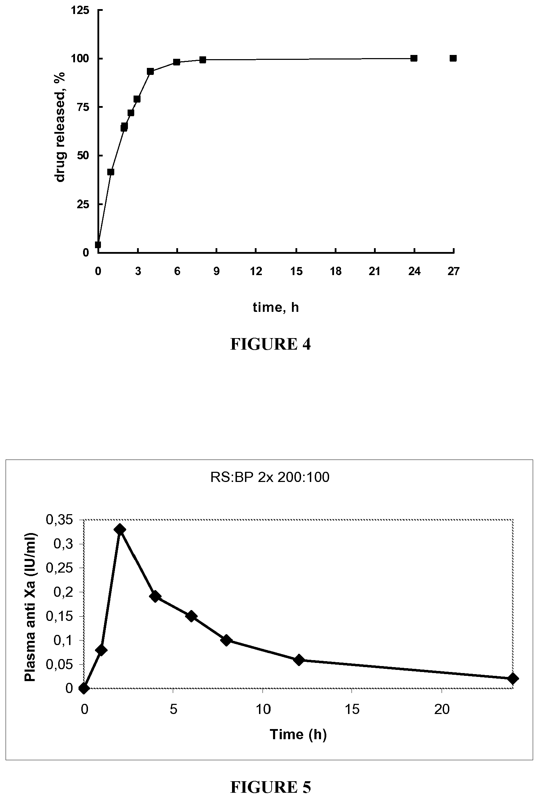

Bemiparin release profile from tablets obtained in this example is shown in Figure 4. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to two tablets per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 5. This Eudragit® RSPO proportion gives rise to a approximately 0.21 µmol ammonium units per milligram of formulation.

### Example 3: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 200 |
| Bemiparin | 200 |
| Mg Stearate | 2 |

This Eudragit® RSPO proportion gives rise to a range of 0.130-0.196 (approximately mean of 0.16) µmol ammonium units per milligram of formulation. Ammonium unit was considered as the ammonium methacrylate monomer present on Eudragit® RSPO and not as its hydrochloride salt.

Humidity or moisture content of the present tablet composition was determined by weight loss on drying at 100°C during 15min. The mean value of humidity was 3.89, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

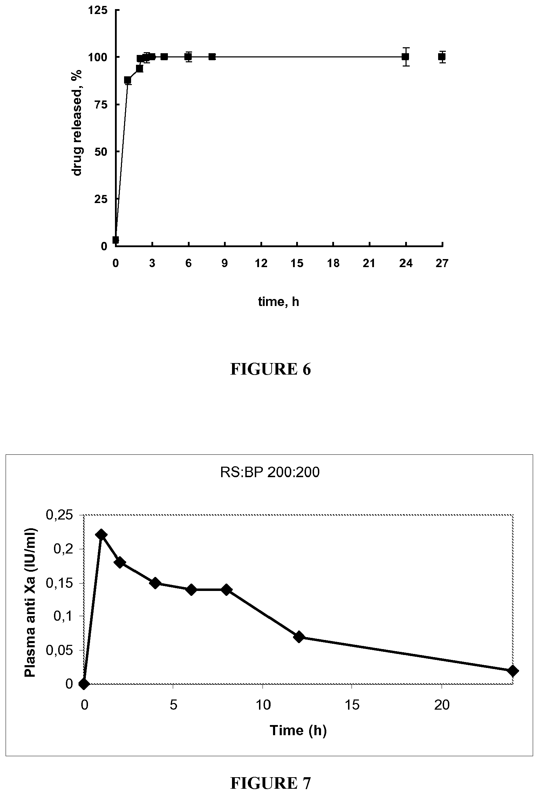

Bemiparin release profile from tablets obtained in this example is shown in Figure 6. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 7. This Eudragit® RSPO proportion gives rise to a approximately 0.16 µmol ammonium units per milligram of formulation.

### Example 4: Bemiparin tablets

Bemiparin tablets were obtained by direct compression of ingredients in powder by using a single punch tablet press. Composition of the powder mixture, per tablet, was the following:

| Ingredient | Amount (mg) |
|---|---|
| Eudragit® RS PO | 800 |
| Bemiparin | 200 |
| Mg Stearate | 5 |

This Eudragit® RSPO proportion gives rise to a range of 0.207-0.313 (approximately mean of 0.26) µmol ammonium units per milligram of formulation. Ammonium unit was considered as the ammonium methacrylate monomer present on Eudragit® RSPO and not as its hydrochloride salt.

Humidity content of the tablet corresponding to this example composition was determined by weight loss on drying at 100°C during 15min. The mean value of humidity was 7.85, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

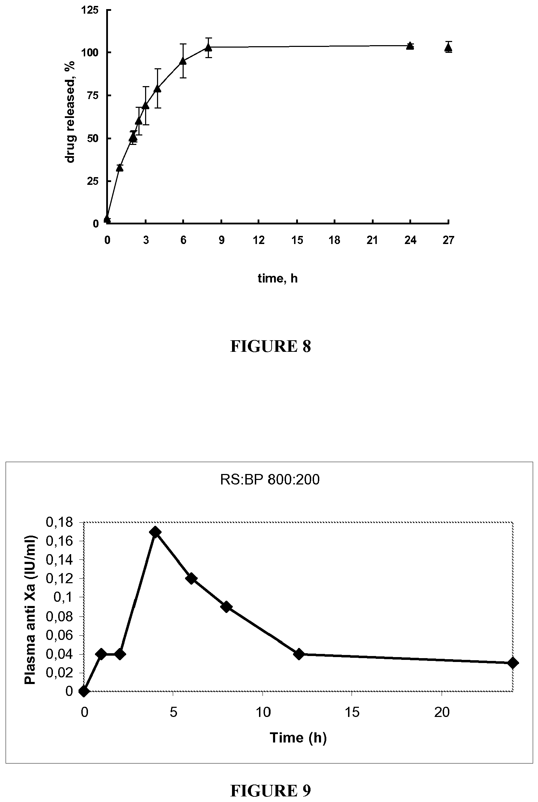

Bemiparin release profile from tablets obtained in this example is shown in Figure 8. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 9. This Eudragit® RSPO proportion gives rise to a approximately 0.26 µmol ammonium units per milligram of formulation.

### Example 5: Bemiparin tablets

Bemiparin tablets were obtained by direct compression of ingredients in powder by using a single punch tablet press. Composition of the powder mixture, per tablet, was the following:

| Ingredient | Amount (mg) |
|---|---|
| Eudragit® RSPO | 1000 |
| Bemiparin | 200 |
| Mg Stearate | 6 |

This Eudragit® RSPO proportion gives rise to a range of 0.216-0.326 (approximately mean of 0.27) µmol ammonium units per milligram of formulation. Ammonium unit was considered as the ammonium methacrylate monomer present on Eudragit® RSPO and not as its hydrochloride salt.

Humidity content of the present tablet composition was determined by weight loss on drying at 100°C during 15min. The mean value of humidity was 7.15, expressed as (w/w, %).

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs did result in plasma levels below to 0,1 IU/ml at all sampling points. This Eudragit® RSPO proportion gives rise to a approximately 0.27 µmol ammonium units per milligram of formulation.

As we can see in example 1 to 5, different GAG to polymer ratios have been tested in order to elucidated which proportion provides better mucosal absorption. 1:5 ratio (see example 5) results in a poor absorption of the compound with a plasma levels below to 0,1 IU/ml at all sampling points. However, 1:1 and 1:2 ratios (see examples 1 to 3) results in a good mucosal absorption, with a plasma levels over 0,20 UI/ml and over 0,1 IU/ml for ratio 1:4 (example 4).

### Example 6: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RL | 400 |
| Bemiparin | 200 |
| Mg Stearate | 3 |

This Eudragit® RLPO proportion give rise to a range 0.341-0.461 (approximately mean of 0.40) µmol ammonium units per milligram of formulation. Ammonium unit was considered as the ammonium methacrylate monomer present on Eudragit® RLPO and not as its hydrochloride salt.

Humidity content of the tablet corresponding to this example composition was determined by weight loss on drying at 100°C during 15min. The mean value of humidity was 3.92, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

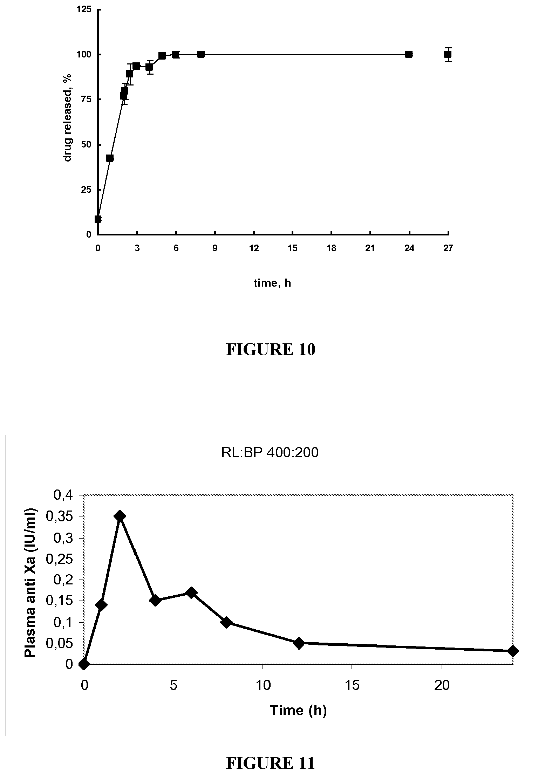

Bemiparin release profile from tablets obtained in this example is shown in Figure 10. Results are expressed as % Bemiparin released from tablets as function of time. This Eudragit® RSPO proportion give rise to a approximately 0.4 µmol ammonium units per milligram of formulation.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 11. This Eudragit® RL proportion gives rise to a approximately 0.4 µmol ammonium units per milligram of formulation.

### Comparative Example 7: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® E | 400 |
| Bemiparin | 200 |
| Mg Stearate | 3 |

This Eudragit® E proportion gives rise to an approximately mean of 2.12 µmol ammonium units per milligram of formulation. Ammonium unit was considered as the dimethyl amino-ethyl group present on Eudragit® E polymer.

Humidity content of the present tablet composition was determined by weight loss on drying at 100°C during 15min. The mean value of humidity was 6.19, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

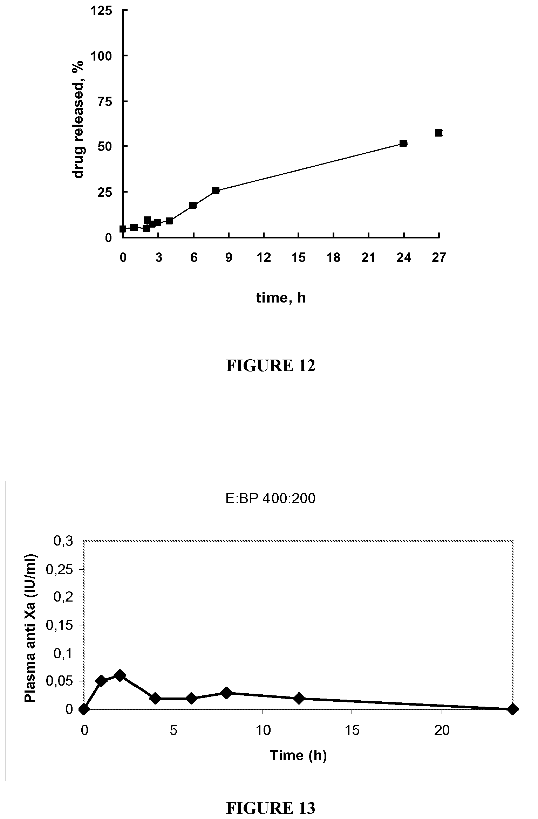

Bemiparin release profile from tablets obtained in this example is shown in Figure 12. Results are expressed as % Bemiparin released from tablets as function of time.

As can be seen in the figure, Eudragit® E strongly complexes Bemiparin and release in 24 h is about 50%. Even if tablet is mechanically disintegrated (point 27h) no more than 60% of the total Bemiparin becomes dissolved in the release medium.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 13. This Eudragit® E proportion gives rise to a approximately 2.1 µmol ammonium units per milligram of formulation.

### Comparative Example 8: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® L | 400 |
| Bemiparin | 200 |
| Mg Stearate | 3 |

Composition presented in this example has no component providing ammonium groups. Humidity content of the tablet corresponding to this example composition was determined by weight loss on drying at 100°C during 15min. The mean value of humidity was 4.26, expressed as (w/w, %).

### In nitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

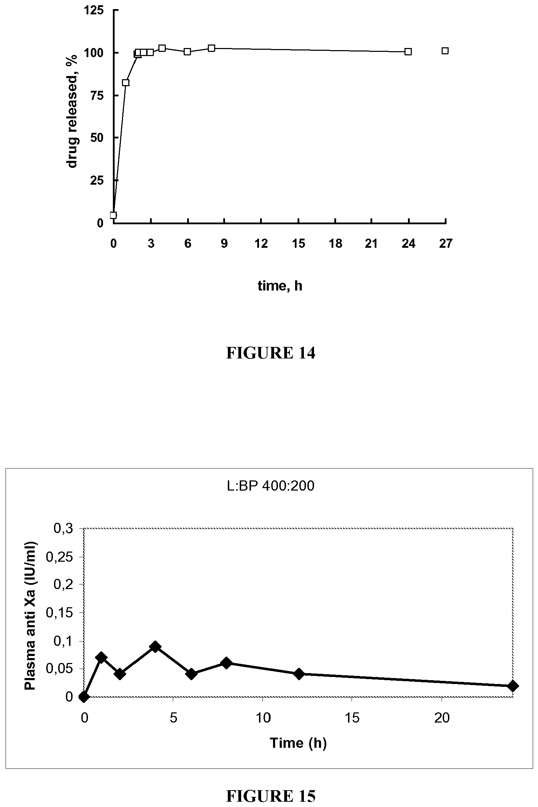

Bemiparin release profile from tablets obtained in this example is shown Figure 14. Results are expressed as % Bemiparin released from tablets as function of time.

As can be seen in the figure, Eudragit® L does not avoid rapid diffusion of Bemiparin through the polymer. This is probably due to the relatively high percentage of Bemiparin in the polymer which helps formation of pores in the structure of the insoluble Eudragit® L polymer.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in detectable plasma levels as shown in Figure 15.

As we can see in example 6 to 8, different types of polymers have been tested in order to determine the influence of the amount of charges and the nature of the charged compound GAG mucosal absorption (all these polymers have similar chemical structures but different ammonium proportions). In all the assays GAG/polymer ratio is 1:2.

Figures 11, 13 and 15 represented plasma activity values obtained when Eudragit® RL, Eudragit® E and Eudragit® L are tested, respectively.

Eudragit® RL (aprox. 0,4 µmol Ammonium units/mg formulation) gives the best results reaching a plasma activity of 0,35 UI/ml.

Eudragit® E (aprox. 2,12 µmol ammonium units/mg formulation) gives a poor plasma activity reaching the maximum value at 0,06 IU/ml.

Eudragit® L, a polymer without ammonium groups, gives the worst results with poor absorption (0,09 IU/ml) and irregular levels.

To sump up these results we can conclude that the best ammonium proportion for our pharmaceutical formulation should be below 2 µmol ammonium units/mg formulation.

### Example 9: Bemiparin minitablets

Bemiparin minitablets were obtained by direct compression of ingredients in powder by using a 2 mm diameter punch in a minitablet press. Composition of the powder mixture, per minitablet, was the following:

| Ingredient | Amount (mg) |
|---|---|
| Eudragit® RS PO | 5 |
| Bemiparin | 2.5 |
| Mg Stearate | 0.05 |
| Fumed silica | 0.05 |

This Eudragit® RS proportion gives rise to a range of 0.173-0.261 (approximately mean of 0,21) µmol ammonium units per milligram of formulation. Ammonium unit was considered as the ammonium methacrylate monomer present on Eudragit® RSPO and not as its hydrochloride salt.

Humidity content of the present minitablet composition was determined by weight loss on drying at 100°C during 15min. The mean value of humidity was 3.44, expressed as (w/w, %).

### In vitro release profile:

Bemiparin release from minitablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry.

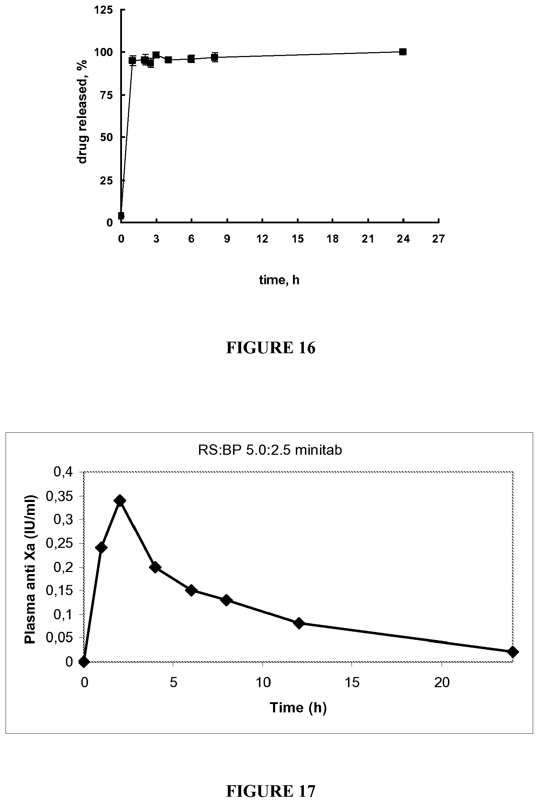

Bemiparin release profile from minitablets obtained in this example is shown in Figure 16. Results are expressed as % Bemiparin released from minitablets as function of time.

### In vivo plasma levels after oral administration to Beagle dog

Bemiparin minitablets obtained in this example were orally given to Beagle dogs weighing an average of 15 kg. The dose was 20000 IU Bemiparin. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in minitablets to Beagle dogs resulted in detectable plasma levels as shown in Figure 17. This Eudragit® RS proportion gives rise to a approximately 0,21 µmol ammonium units per milligram of formulation.

### Example 10: Bemiparin minitablets

Bemiparin minitablets were obtained by direct compression of ingredients in powder by using a 2 mm diameter punch in a minitablet press. Composition of the powder mixture, per minitablet, was the following:

| Ingredient | Amount (mg) |
|---|---|
| Eudragit® RS PO | 3.75 |
| Bemiparin | 3.75 |
| Mg Stearate | 0.04 |
| Fumed silica | 0.04 |

This Eudragit® RS proportion gives rise to a range of 0.130-0,196 (approximately mean of 0.16) µmol ammonium units per milligram of formulation. Ammonium unit was considered as the ammonium methacrylate monomer present on Eudragit® RSPO and not as its hydrochloride salt.

Humidity content of the tablet corresponding to this example composition was determined by weight loss on drying at 100°C during 15min. The mean value of humidity was 2.56, expressed as (w/w, %).

### In vitro release profile:

Bemiparin release from minitablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry.

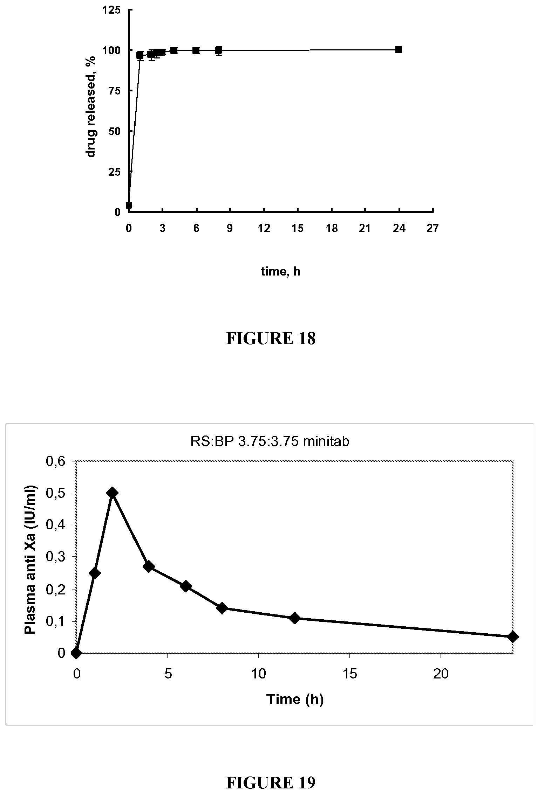

Bemiparin release profile from minitablets obtained in this example is shown in Figure 18 (results are expressed as % Bemiparin released from minitablets as function of time):

### In vivo plasma levels after oral administration to Beagle dog

Bemiparin minitablets obtained in this example were orally given to Beagle dogs weighing an average of 15 kg. The dose was 20000 IU Bemiparin. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in minitablets to Beagle dogs resulted in detectable plasma levels as shown in Figure 19.

### Example 11: Bemiparin minitablets

Bemiparin minitablets were obtained by direct compression of ingredients in powder by using a 2 mm diameter punch in a minitablet press. Composition of the powder mixture, per minitablet, was the following:

| Ingredient | Amount (mg) |
|---|---|
| Eudragit® RS PO | 6.0 |
| Bemiparin | 1.5 |
| Mg Stearate | 0.04 |
| Fumed silica | 0.04 |

This Eudragit® RSPO proportion gives rise to a range of 0.207-0.313 (approximately mean of 0.26) umol ammonium units per milligram of formulation. Ammonium unit was considered as the ammonium methacrylate monomer present on Eudragit® RSPO and not as its hydrochloride salt.

Humidity content of the present minitablet composition was determined by weight loss on drying at 100°C during 15min. The mean value of humidity was 3.79, expressed as (w/w, %).

### In vitro release profile:

Bemiparin release from minitablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h minitablets were disintegrated in the release medium with a homogenizer and the whole content remained 10 min to determine maximum release of Bemiparin from the sample. In order to clearly represent this value in the graph the data point has been plotted at 27 h.

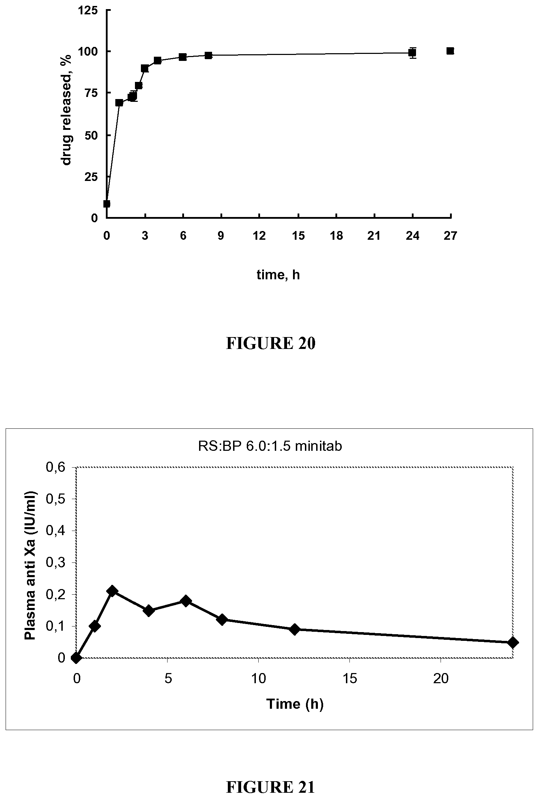

Bemiparin release profile from minitablets obtained in this example is shown in Figure 20. Results are expressed as % Bemiparin released from minitablets as function of time.

### In vivo plasma levels after oral administration to Beagle dog

Bemiparin minitablets obtained in this example were orally given to Beagle dogs weighing an average of 15 kg. The dose was 20000 IU Bemiparin. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in minitablets to Beagle dogs resulted in detectable plasma levels as shown in Figure 21.

As we can see in examples 9 to 11 with the same polymer (Eudragit® RS), GAG to polymers ratios have been tested in order to elucidated which proportion provides better mucosal absorption Bemiparin:Eudragit 1:4 ratio (see example 11) results in a absorption of the compound with a plasma levels over to 0,2 IU/ml. However, 1:2 and 1:1 ratios (see examples 9 to 10) results in a good mucosal absorption, with a maximum plasma levels since 0,35 UI/ml to 0,5 UI/ml.

In connection with examples 1 to 5 (tablets), for our pharmaceutical form 1:1 and 1:2 ratios (see examples 1, 3, 6, 9 and 10) are preferred because results in a good mucosal absorption, with a plasma levels over 0,20 UI/ml.

Therefore, for our pharmaceutical form, the percentage of GAG in the formulation is between 20% to 50% w/w.

### Example 12: Fondaparinux minitablets

Fondaparinux minitablets were obtained by direct compression of ingredients in powder by using a 2 mm diameter punch in a minitablet press. Composition of the powder mixture, per minitablet, was the following:

| Ingredient | Amount (mg) |
|---|---|
| Fondaparinux | 2.5 |
| Eudragit® RSPO | 5.0 |
| Mg stearate | 0.04 |

### In vivo plasma levels after intragastric administration to Wistar rats

Fondaparinux minitablets obtained in this example were intragastrically given to Wistar rats weighing an average of 300 g. The dose was 2.5 mg Fondaparinux. Total number of rats was 6. After intragastric administration, plasma samples were obtained at 0, 0.5, 1, 2 and 4 hours and Fondapainux absorption was evaluated by measuring plasma anti factor Xa activity in the samples. Results are expressed as mg/L Fondaparinux in plasma.

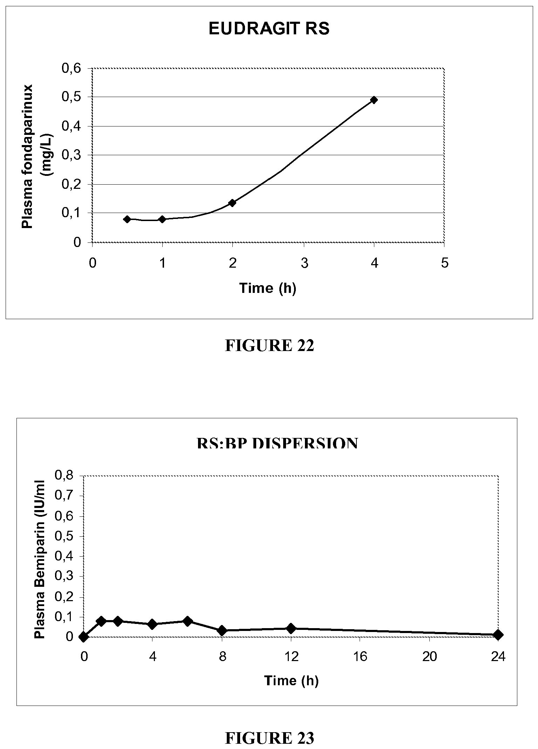

Intragastric administration of 2.5 mg Fondaparinux in minitablets to Wistar rats resulted in detectable plasma levels as shown in Figure 22.

Plasma anti Xa activity in Wistar rats after intragastric administration of 2.5 mg fondaparinux tablets obtained in this example [glycosaminoglycan (fondaparinux) /compound presenting quaternary ammonium groups (Eudragit® RSPO)=2.5mg/5.0mg]. This Eudragit® RSPO proportion gives rise to an approximately mean of 0.22 µmol ammonium units per milligram of formulation.

This example has been carried out to check if a compound containing ammonium groups attached to a non water soluble scaffold can improved mucosal oral absorption of a glycosaminoglycan (GAG).

Eudragit® RS was selected has a good candidate due to its cationic nature (this polymer contains trimethylated ammonium groups) and absence of water solubility. A priori, trimethyl ammonium groups also seem to be a good choice since distance between sulphate groups in the GAG studied is long enough to fit them (3.5 Å).

Results are expressed as mg/L Fondaparinux in plasma.

Trimethyl ammonium combined with a non water soluble sacaffold gives significantly better results than the previous combinations since fondaparinux plasma levels reach 0.5 mg/L in four hours.

### Comparative example 13: Bemiparin in Eudragit® RS dispersion

A Bemiparin formulation in Eudragit® RS dispersion was prepared as follows. First, the required amount of Bemiparin and Eudragit® RS was dissolved in water. Composition of the formulation, per 200 mg Bemiparin dose, was the following:

| Ingredient | Amount (mg) |
|---|---|
| Bemiparin | 200 |
| Water | 350 |
| Eudragit® RS | 200 |

This Eudragit® RS proportion gives rise to a range of 0.069-0.105 (approximately mean of 0,09) µmol ammonium units per milligram of formulation.

This example has been carried out to check if a non solid matrix pharmaceutical form can improve mucosal oral absorption of a glycosaminoglycan (GAG).

As can be observed, same composition regarding GAG and polymer nature and quantities formulated as a solid matrix (tablets and minitablets) reached plasma levels over 0.3 UI/ml. On the other hand, example 13 formulation presented as a non solid form resulted in plasma levels below 0.1 UI/ml.

The presence of ammonium charges on the surface of a solid form improve GAG oral absorption. The GAG oral absorption is also improved for the fact that these ammoniums are located in an structured scaffold, which seems to be a key point to notably improve GAG oral absorption.

### In vivo plasma levels after oral administration to Beagle dogs

Bemiparin formulations obtained in this example were orally given to Beagle dogs weighing an average of 15 kg. The dose was 20000 IU Bemiparin per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in a formulation according to this example to Beagle dogs resulted in very low plasma levels as shown in Figure 23. This Eudragit® RS proportion gives rise to a approximately 0,1 µmol ammonium units per milligram of formulation.

This example has been carried out to check if a non solid matrix pharmaceutical form can improve mucosal oral absorption of a glycosaminoglycan (GAG).

As can be observed, same composition regarding GAG and polymer nature and quantities (see example 3 and 10) formulated as a solid matrix (tablets and minitablets) reached plasma levels over 0.3 UI/ml. On the other hand, example 13 formulation presented as a non solid form resulted in plasma levels below 0.1 UI/ml.

### Example 14: Bemiparin tablets

Bemiparin tablets were obtained by direct compression of ingredients in powder by using a single punch tablet press. Composition of the powder mixture, per tablet, was the following:

| Ingredient | Amount (mg) |
|---|---|
| Eudragit® RS PO | 400 |
| Bemiparin | 200 |
| Mg Stearate | 3 |

This Eudragit® RSPO proportion gives rise to a range of 0.207-0.313 (approximately mean of 0.26) µmol ammonium units per milligram of formulation Ammonium unit was considered as the ammonium methacrylate monomer present on Eudragit® RSPO and not as its hydrochloride salt.

Humidity content of the tablet corresponding to this example composition was determined by weight loss on drying at 100°C during 15min. The mean value of humidity was 12.07, expressed as (w/w, %).

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

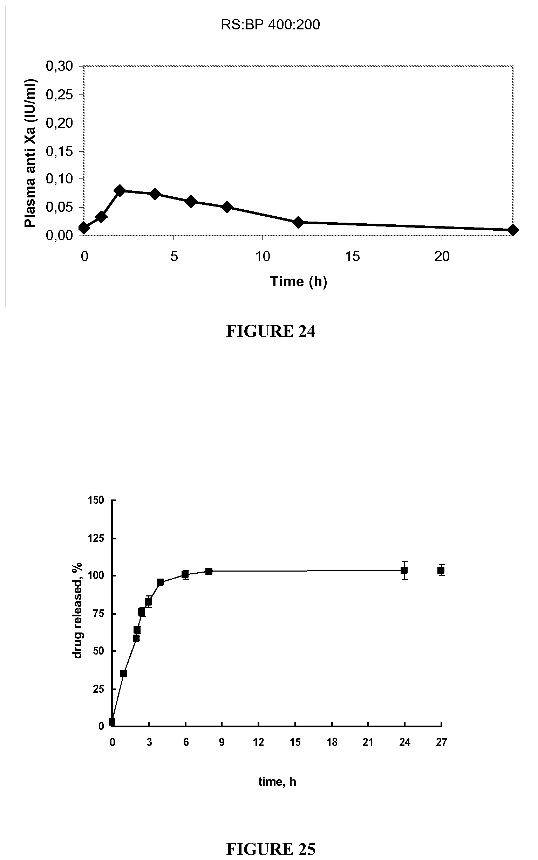

Oral administration of 20000 IU Bemiparin in tablets to Beagle dogs resulted in detectable plasma levels below to 0,1 IU/ml at all sampling points as shown in Figure 24.

As we can see in example 2, a composition with a same GAG to polymer ratio and presence of ammonium groups (expressed as µmol/mg of formulation) than in example 16, but with different humidity (w/w, %), has been tested in order to elucidated an influence of this parameter on mucosal absorption. Same composition with a humidity of 5.79 % (Figure 3, example 2) results in a good mucosal absorption with plasma levels over 0,20, whereas that composition with a humidity content of 12.07 % results in a poor absorption of the compound with a plasma levels below to 0,1 IU/ml at all sampling points. Hence, different compositions described on example 1 to 4, and 9 to 11, all of then corresponding to humidity contents values in range 2.56-7.85 % elicit substantial GAG mucosal absorption with plasma levels over 0.15 IU/ml.

To sump up these results we can conclude that the best humidity content for our pharmaceutical formulation should be below 12 % (w/w).

### Example 15: Enoxaparin tablets

Enoxaparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 400 |
| Enoxaparin | 200 |
| Mg Stearate | 3 |

This Eudragit® RSPO proportion give rise to a range of 0.173-0.261 (approximately mean of 0.21) µmol ammonium units per milligram of formulation. Ammonium unit was considered as the ammonium methacrylate monomer present on Eudragit® RSPO and not as its hydrochloride salt.

### In nitro release profile:

Enoxaparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Enoxaparin amount present was determined by nephelometry. After 24 h tablets were disintegrated in the release medium with a homogenizer and the whole content remained 10 min to determine maximum release of Enoxaparin from the sample. In order to clearly represent this value in the graph the data point has been plotted at 27 h.

Enoxaparin release profile from tablets obtained in this example is shown in Figure 25. Results are expressed as % Enoxaparin released from tablets as function of time.

### In vivo plasma levels after oral administration to Beagle dog

Enoxaparin tablets obtained in this example were orally given to Beagle dogs weighing an average of 15 kg. The dose was 20000 IU Enoxaparin that corresponded to one single tablet per animal. Total number of dogs was 3. After oral administration, plasma samples were obtained at 0, 4, 8, 12, 18 and 24 hours and Enoxaparin absorption was evaluated by measuring plasma anti factor Xa activity in the samples.

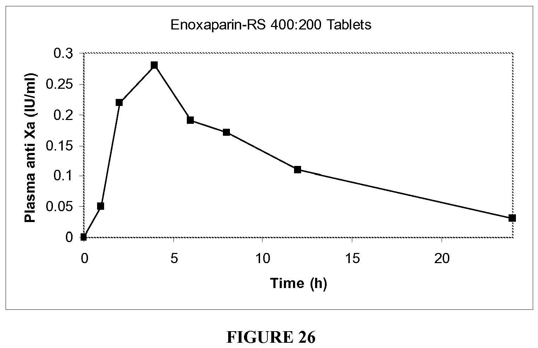

Oral administration of 20000 IU Enoxaparin in tablets to Beagle dogs resulted in good mucosal absortion reaching plasma levels of 0.28 IU/ml (Figure 26).

### Example 16: Enoxaparin tablets

Enoxaparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 200 |
| Enoxaparin | 200 |
| Mg Stearate | 2 |

This Eudragit® RSPO proportion gives rise to a range of 0.130-0.196 (approximately mean of 0.16) µmol ammonium units per milligram of formulation. Ammonium unit was considered as the ammonium methacrylate monomer present on Eudragit® RSPO and not as its hydrochloride salt.

### In vitro release profile:

Enoxaparin® release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Enoxaparin amount present was determined by nephelometry. After 24 h tablets were disintegrated in the release medium with a homogenizer and the whole content remained 10 min to determine maximum release of Enoxaparin from the sample. In order to clearly represent this value in the graph the data point has been plotted at 27 h.

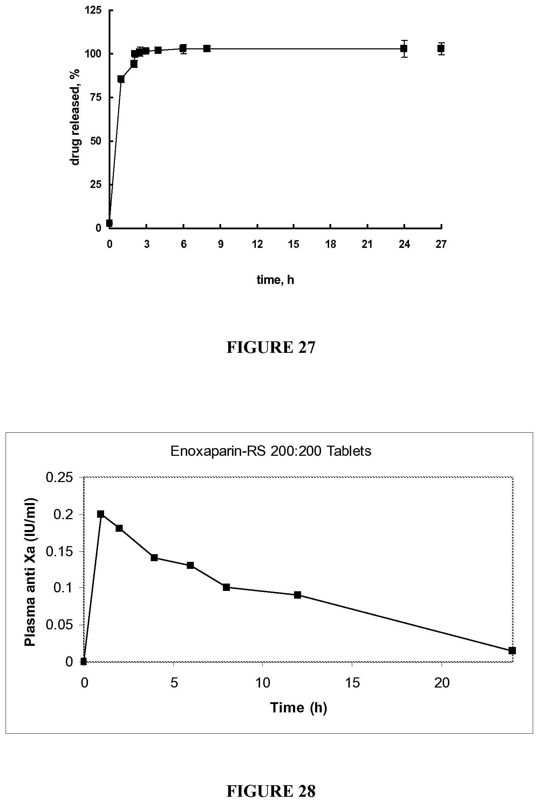

Enoxaparin release profile from tablets obtained in this example is shown in Figure 27. Results are expressed as % Enoxaparin released from tablets as function of time.

### In vivo plasma levels after oral administration to Beagle dog

Enoxaparin tablets obtained in this example were orally given to Beagle dogs weighing an average of 15 kg. The dose was 20000 IU Enoxaparin that corresponded to one single tablet per animal. Total number of dogs was 3. After oral administration, plasma samples were obtained at 0, 4, 8, 12, 18 and 24 hours and enoxaparin absorption was evaluated by measuring plasma anti factor Xa activity in the samples.

Oral administration of 20000 IU Enoxaparin in tablets to Beagle dogs resulted in plasma levels up to 0.2 IU/ml as shown in Figure 28.

### Example 17: Enoxaparin minitablets

Enoxaparin minitablets were obtained by direct compression of ingredients in powder by using a 2 mm diameter punch in a minitablet press. Composition of the powder mixture, per minitablet, was the following:

| Ingredient | Amount (mg) |
|---|---|
| Eudragit® RS PO | 5 |
| Enoxaparin | 2.5 |
| Mg Stearate | 0.05 |
| Fumed silica | 0.05 |

This Eudragit® RS proportion gives rise to a range of 0.173-0.261 (approximately mean of 0,21) µmol ammonium units per milligram of formulation. Ammonium unit was considered as the ammonium methacrylate monomer present on Eudragit® RSPO and not as its hydrochloride salt.

### In vitro release profile:

Enoxaparin release from minitablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Enoxaparin amount present was determined by nephelometry.

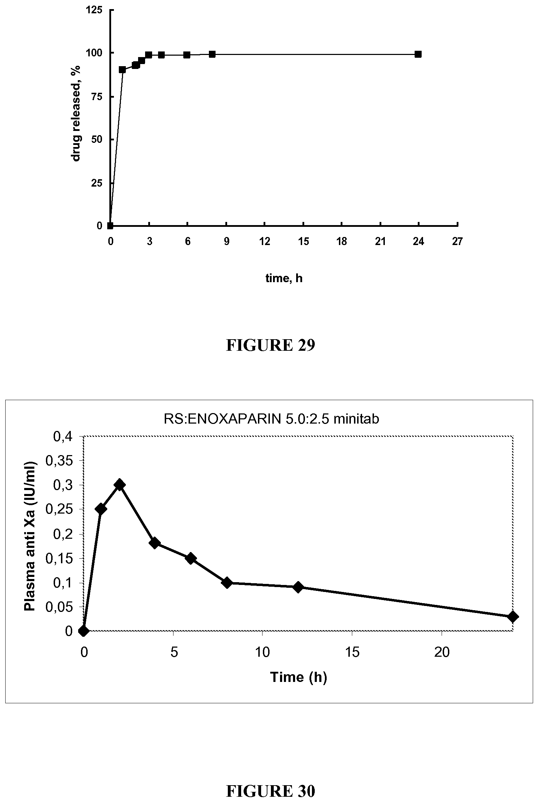

Enoxaparin release profile from minitablets obtained in this example is shown in Figure 29. Results are expressed as % Enoxaparin released from minitablets as function of time.

### In vivo plasma levels after oral administration to Beagle dog

Enoxaparin minitablets obtained in this example were orally given to Beagle dogs weighing an average of 15 kg. The dose was 20000 IU Enoxaparin. Total number of dogs was 3. After oral administration, plasma samples were obtained at 0, 4, 8, 12, 18 and 24 hours and enoxaparin absorption was evaluated by measuring plasma anti factor Xa activity in the samples.

Oral administration of 20000 IU Enoxaparin in minitablets to Beagle dogs resulted in good enoxaparin mucosal absorption with plasma levels up to 0.3 IU/ml as shown in Figure 30.

### Example 18: Enoxaparin minitablets

Enoxaparin minitablets were obtained by direct compression of ingredients in powder by using a 2 mm diameter punch in a minitablet press. Composition of the powder mixture, per minitablet, was the following:

| Ingredient | Amount (mg) |
|---|---|
| Eudragit® RS PO | 3.75 |
| Enoxaparin | 3.75 |
| Mg Stearate | 0.04 |
| Fumed silica | 0.04 |

This Eudragit® RSPO proportion gives rise to a range of 0.130-0.196 (approximately mean of 0.16) µmol ammonium units per milligram of formulation. Ammonium unit was considered as the ammonium methacrylate monomer present on Eudragit® RSPO and not as its hydrochloride salt.

### In nitro release profile:

Enoxaparin release from minitablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Enoxaparin amount present was determined by nephelometry.

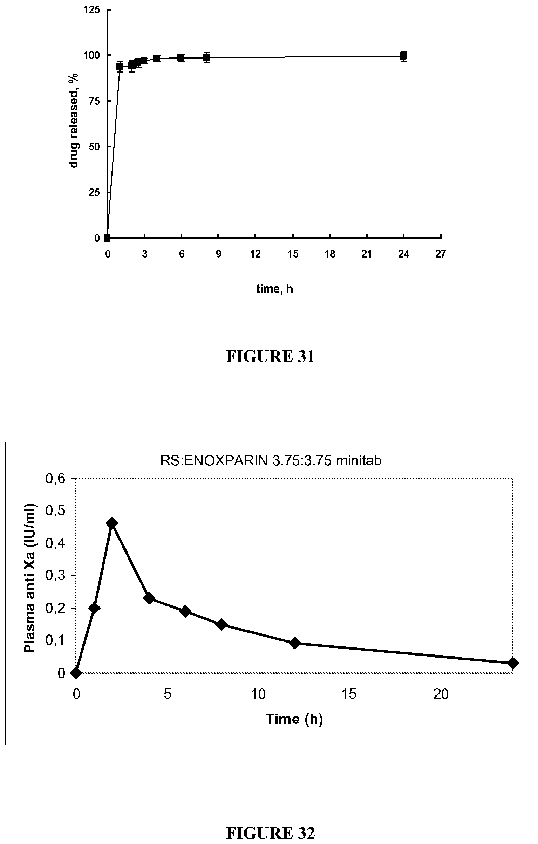

Enoxaparin release profile from minitablets obtained in this example is shown in Figure 31 (results are expressed as % Enoxaparin released from minitablets as function of time):

### In vivo plasma levels after oral administration to Beagle dog

Enoxaparin minitablets obtained in this example were orally given to Beagle dogs weighing an average of 15 kg. The dose was 20000 IU Enoxaparin. Total number of dogs was 3. After oral administration, plasma samples were obtained at 0, 4, 8, 12, 18 and 24 hours and enoxaparin absorption was evaluated by measuring plasma anti factor Xa activity in the samples.

Oral administration of 20000 IU Enoxaparin in minitablets to Beagle dogs resulted in good compound absorption with levels over 0.45 IU/ml (Figure 32).

### Example 19: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 400 |
| Bemiparin | 200 |

This Eudragit® RSPO proportion gives rise to a range of 0.174-0.262 (approximately mean of 0.22) µmol ammonium units per milligram of formulation. Ammonium unit was considered as the ammonium methacrylate monomer present on Eudragit® RSPO and not as its hydrochloride salt.

Humidity content of the present tablet composition was determined by weight loss on drying at 100°C during 15min. The mean value of humidity was 4.03, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

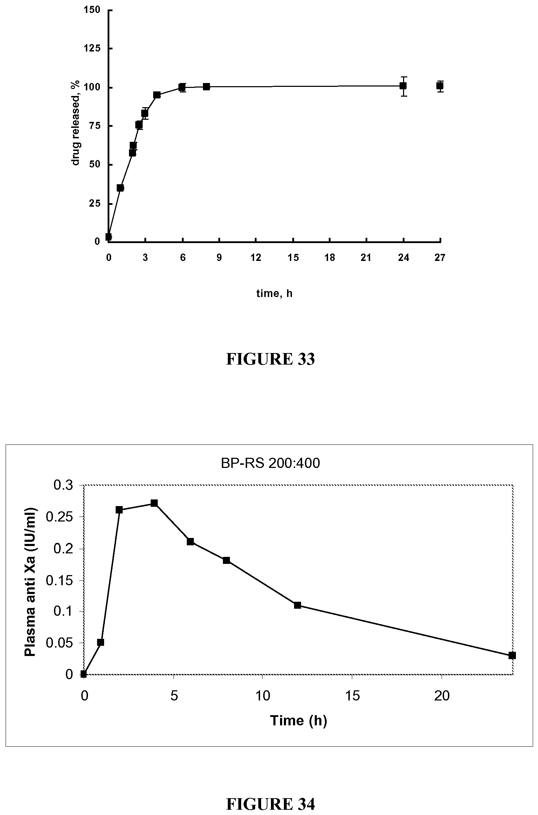

Bemiparin release profile from tablets obtained in this example is shown in Figure 33. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral adminstration of 20000 IU Bemiparin in tablets to Beagle dogs resulted in good compound absorption with levels over 0.2 IU/ml (Figure 34).

### Example 20: Bemiparin tablets

Bemiparin tablets were obtained by direct compression of ingredients in powder by using a 10 mm diameter punch in a single punch tablet press. Composition of the powder mixture, per tablet, was the following:

| Ingredient | Amount (mg) |
|---|---|
| Eudragit® RS PO | 160 |
| Eudragit® E | 410 |
| Bemiparin | 200 |
| Dispersantt | 24 |
| Mg Stearate | 4 |

This Eudragit® RSPO and Eudragit® E proportion gives rise to a range of 1.536-1.899-(approximately mean of 1.72) µmol ammonium units per milligram of formulation. Ammonium unit was considered as the ammonium methacrylate monomer present on Eudragit® RSPO and not as its hydrochloride salt, and as dimethylaminoethyl groups for Eudragit® E.

Humidity content of the present tablet composition was determined by weight loss on drying at 100°C during 15min. The mean value of humidity was 5.13, expressed as (w/w, %).

### In vitro release profile:

Bemiparin release from tablets was evaluated in USP paddle apparatus (Vankel VK 300, Vankel Industries, Edison, NJ, USA) at 37 °C, 100 rpm, n=2. Release medium was 750 ml 0.1 N HCl for 2 h followed by addition of 225 ml of 0.2M trisodium phosphate to adjust pH = 6.8. At previously scheduled time points (1 h, 2 h, 2h 5min, 2 h 30 min, 3 h, 4 h, 6 h, 8 h and 24 h) 3 ml were collected and Bemiparin amount present was determined by nephelometry. After 24 h tablets were disintegrated in the release medium with a homogenizer and the whole content remained 10 min to determine maximum release of Bemiparin from the sample. In order to clearly represent this value in the graph the data point has been plotted at 27 h.

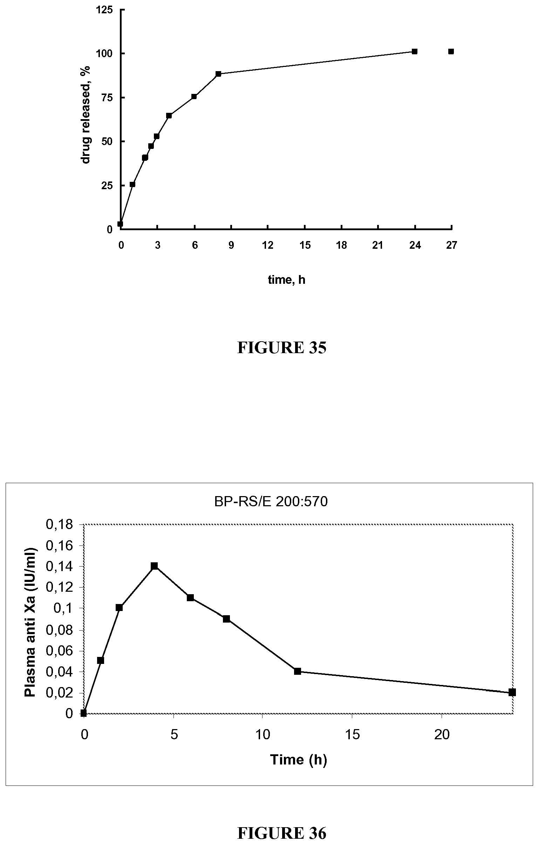

Bemiparin release profile from tablets obtained in this example is shown in Figure 35. Results are expressed as % Bemiparin released from tablets as function of time.

### In vivo plasma levels after oral administration to Beagle dog

Bemiparin tablets obtained in this example were orally given to Beagle dogs weighing an average of 15 kg. The dose was 20000 IU Bemiparin that corresponded to one single tablet per animal. Total number of dogs was 3. After oral administration, plasma samples were obtained at 0, 4, 8, 12, 18 and 24 hours and bemiparin absorption was evaluated by measuring plasma anti factor Xa activity in the samples.

Oral administration of 20000 IU Bemiparin in tablets to Beagle dogs resulted in moderate compound absorption with levels between 0.1-0.2 IU/ml (Figure 36).

## Claims

1. A pharmaceutical form comprising a glycosaminoglycan selected from the group consisting of: bemiparin, fondaparinux and enoxaparin and its pharmaceutically acceptable salts; and a compound presenting independent or dependent pH quaternary ammonium groups which is a structure of polymers and copolymers derived from acrylic and methacrylic acids esters , wherein:
- the proportion of ammonium groups in the pharmaceutical form is between 0.01 to 2.0 µmol ammonium/mg pharmaceutical form,
- the proportion of glycosaminoglycan in the pharmaceutical form is between 15 % to 50% w/w, and
- the pharmaceutical form presents a humidity percentage (w/w) lower than 10%, wherein the charges of the glycosaminoglycan are only partially neutralized with selected cations which are the dependent or independent pH quaternary ammonium groups.

2. The pharmaceutical form according to any of the claims 1 wherein the compound presenting dependent or independent pH quaternary ammonium groups is a non water soluble compound.

3. The pharmaceutical form according to any of the claims 1 to 2 wherein the proportion of ammonium groups in the pharmaceutical form is between 0.05-1.7 µmol ammonium/mg pharmaceutical form.

4. The pharmaceutical form according to claim 3, wherein the proportion of ammonium groups in the pharmaceutical form is between 0.21-0.4 µmol ammonium/mg pharmaceutical form.

5. The pharmaceutical form according to any of the claims 1 to 4 wherein the proportion of glycosaminoglycan in the pharmaceutical form is between 25-50% w/w.

6. The pharmaceutical form according to claim 5, wherein the proportion of glycosaminoglycan in the pharmaceutical form is between 33-50% w/w.

7. The pharmaceutical form according to any of the claims 1 to 6, wherein the pharmaceutical form presents a humidity percentage (w/w) lower than 8%.

8. The pharmaceutical form according to any of the claims 1 to 2 wherein,
- the proportion of ammonium groups in the pharmaceutical form is between from about 0.16 to 1.7 µmol ammonium/mg pharmaceutical composition, and
- the proportion of glycosaminoglycan in the pharmaceutical composition is from 20 % to 50% w/w.

9. The pharmaceutical form according to any of the claims 1 to 8 further comprising pharmaceutically acceptable excipients, wherein the percentage (w/w) of excipients or carriers in the formulation is equal or lower than 10%.

10. A pharmaceutical formulation comprising the pharmaceutical form as defined in claim 9, wherein the compound presenting quaternary ammonium groups form part of the surface of the said formulation.

11. The pharmaceutical formulation according to any of the claims 9 to 10, wherein said formulation is a pellet, a granule, a tablet or a minitablet.

12. The pharmaceutical formulation according to any of the claims 9 to 11, wherein said formulation is for administration by oral route.

13. The pharmaceutical formulation according to claim 12, wherein said formulation is a non extended release formulation.

14. A pharmaceutical form as defined in any of the claims 1 to 8 or a pharmaceutical formulation as defined in any of the claims 9 to 13, for use as a medicament.

## Patentansprüche

1. Pharmazeutische Form, umfassend ein Glykosaminoglykan, das ausgewählt wird aus der Gruppe bestehend aus: Bemiparin, Fondaparinux und Enoxaparin sowie deren pharmazeutisch annehmbaren Salzen; und eine Verbindung mit pH-unabhängigen oder pH-abhängigen quaternären Ammoniumgruppen, die eine Struktur aus von Acryl- und Methacrylsäureestern abgeleiteten Polymeren und Copolymeren darstellt, wobei:
- der Anteil an Ammoniumgruppen in der pharmazeutischen Form zwischen 0,01 und 2,0 µmol Ammonium/mg pharmazeutischer Form liegt,
- der Anteil an Glykosaminoglykan in der pharmazeutischen Form zwischen 15% und 50 % Gew./Gew. liegt, und
- die pharmazeutische Form einen Feuchtigkeitsgehalt (Gew./Gew.) unter 10 % aufweist, wobei die Ladungen des Glykosaminoglykans nur teilweise mit ausgewählten Kationen neutralisiert werden, die die pH-abhängigen oder pH-unabhängigen quaternären Ammoniumgruppen sind.

2. Pharmazeutische Form nach einem der Ansprüche 1, wobei die Verbindung mit den pH-abhängigen oder pH-unabhängigen quaternären Ammoniumgruppen eine nicht wasserlösliche Verbindung ist.

3. Pharmazeutische Form nach einem der Ansprüche 1 bis 2, wobei der Anteil an Ammoniumgruppen in der pharmazeutischen Form zwischen 0,05 und 1,7 µmol Ammonium/mg pharmazeutischer Form liegt.

4. Pharmazeutische Form nach Anspruch 3, wobei der Anteil an Ammoniumgruppen in der pharmazeutischen Form zwischen 0,21 und 0,4 µmol Ammonium/mg pharmazeutischer Form liegt.

5. Pharmazeutische Form nach einem der Ansprüche 1 bis 4, wobei der Anteil an Glykosaminoglykan in der pharmazeutischen Form zwischen 25 und 50 % Gew./Gew. liegt.

6. Pharmazeutische Form nach Anspruch 5, wobei der Anteil an Glykosaminoglykan in der pharmazeutischen Form zwischen 33 und 50 % Gew./Gew. liegt.

7. Pharmazeutische Form nach einem der Ansprüche 1 bis 6, wobei die pharmazeutische Form einen Feuchtigkeitsprozentsatz (Gew./Gew.) unter 8 % aufweist.

8. Pharmazeutische Form nach einem der Ansprüche 1 bis 2, wobei
- der Anteil an Ammoniumgruppen in der pharmazeutischen Form zwischen ungefähr 0,16 und 1,7 µmol Ammonium/mg pharmazeutischer Zusammensetzung liegt und
- der Anteil an Glykosaminoglykan in der pharmazeutischen Zusammensetzung zwischen 20 % und 50 % Gew./Gew. liegt.

9. Pharmazeutische Form nach einem der Ansprüche 1 bis 8, die weiterhin pharmazeutisch annehmbare Hilfsstoffe umfasst, wobei der Prozentsatz (Gew./Gew.) an Hilfsstoffen oder Trägerstoffen in der Formulierung gleich oder kleiner als 10 % ist.

10. Pharmazeutische Formulierung, die die pharmazeutische Form nach Anspruch 9 umfasst, wobei die Verbindung mit quaternären Ammoniumgruppen Teil der Oberfläche dieser Formulierung ist.

11. Pharmazeutische Formulierung nach einem der Ansprüche 9 bis 10, wobei die Formulierung ein Pellet, ein Granulat, eine Tablette oder eine Minitablette ist.

12. Pharmazeutische Formulierung nach einem der Ansprüche 9 bis 11, wobei die Formulierung zur oralen Verabreichung vorgesehen ist.

13. Pharmazeutische Formulierung nach Anspruch 12, wobei die Formulierung eine Formulierung ohne verlängerte Freisetzung ist.

14. Pharmazeutische Form nach einem der Ansprüche 1 bis 8 oder pharmazeutische Formulierung nach einem der Ansprüche 9 bis 13 zur Verwendung als Arzneimittel.

## Revendications

1. Une forme pharmaceutique comprenant un glycosaminoglycane choisi dans le groupe consistant en:
bemiparine, fondaparinux et énoxaparine et ses sels pharmaceutiquement acceptables ; et un composé présentant des groupes d'ammonium quaternaire à pH indépendant ou dépendant qui est une structure de polymères et de copolymères dérivés d'esters d'acides acrylique et méthacrylique,
dans lequel :
- - la proportion de groupes ammonium sous forme pharmaceutique est comprise entre 0,01 et 2,0 µmol d'ammonium / mg de forme pharmaceutique,
- la proportion de glycosaminoglycane sous la forme pharmaceutique est comprise entre 15 % et 50 % p / p, et
- la forme pharmaceutique présente un pourcentage d'humidité (p / p) inférieur à 10 %, dans lequel les charges du glycosaminoglycane ne sont que partiellement neutralisées avec des cations sélectionnés qui sont les groupes d'ammonium quaternaire à pH dépendants ou indépendants.

2. Forme pharmaceutique selon l'une quelconque des revendications 1, dans laquelle le composé présentant des groupes d'ammonium quaternaire à pH dépendants ou indépendants est un composé non soluble dans l'eau.

3. La forme pharmaceutique selon l'une quelconque des revendications 1 à 2, dans laquelle la proportion de groupes ammonium sous la forme pharmaceutique est comprise entre 0,05 - 1,7 µmol d'ammonium / mg de forme pharmaceutique.

4. La forme pharmaceutique selon la revendication 3, dans laquelle la proportion de groupes ammonium sous forme pharmaceutique est comprise entre 0,21 - 0,4 µmol de formule pharmaceutique ammonium / mg.

5. Forme pharmaceutique selon l'une quelconque des revendications 1 à 4, dans laquelle la proportion de glycosaminoglycane sous la forme pharmaceutique est comprise entre 25-50 % p / p.

6. La forme pharmaceutique selon la revendication 5, dans laquelle la proportion de glycosaminoglycane sous la forme pharmaceutique est comprise entre 33 - 50 % p / p.

7. La forme pharmaceutique selon l'une quelconque des revendications 1 à 6, dans laquelle la forme pharmaceutique présente un pourcentage d'humidité (p / p) inférieur à 8 %.

8. La forme pharmaceutique selon l'une quelconque des revendications 1 à 2, dans laquelle,
- - la proportion de groupes ammonium sous forme pharmaceutique se situe entre environ 0,16 à 1,7 µmol de composition ammonium / mg pharmaceutique et
- - la proportion de glycosaminoglycane dans la composition pharmaceutique est de 20 % à 50 % p / p.

9. Forme pharmaceutique selon l'une quelconque des revendications 1 à 8, comprenant en outre des excipients pharmaceutiquement acceptables, dans lequel le pourcentage (p / p) d'excipients ou de supports dans la formulation est égal ou inférieur à 10 %.

10. Une formulation pharmaceutique comprenant la forme pharmaceutique selon la revendication 9, dans laquelle le composé présentant des groupes ammonium quaternaire fait partie de la surface de ladite formulation.

11. Formulation pharmaceutique selon l'une quelconque des revendications 9 à 10, dans laquelle ladite granulation est une pastille, un granulé, un comprimé ou une mini tablette.

12. Formulation pharmaceutique selon l'une quelconque des revendications 9 à 11, dans laquelle ladite formulation est une administration à distance par voie orale.

13. Formulation pharmaceutique selon la revendication 12, dans laquelle ladite formulation est une formulation à libération non étendue.

14. Une ferme pharmaceutique selon l'une quelconque des revendications 1 à 8 ou une ferme pharmaceutique selon l'une quelconque des revendications 9 à 13, très utilisée comme médicament.
